(19) Europäisches Patentamt
European Patent Office
Office européen des brevets

(11) EP 4 008 784 A1

(12) EUROPEAN PATENT APPLICATION
published in accordance with Art. 153(4) EPC

(43) Date of publication:
**08.06.2022 Bulletin 2022/23**

(21) Application number: **20846396.8**

(22) Date of filing: **31.07.2020**

(51) International Patent Classification (IPC):
*C12N 15/11 (2006.01)* *A61K 31/7105 (2006.01)*
*A61K 48/00 (2006.01)* *C12N 1/15 (2006.01)*
*C12N 1/19 (2006.01)* *C12N 1/21 (2006.01)*
*C12N 5/10 (2006.01)* *C12N 9/78 (2006.01)*
*C12N 15/09 (2006.01)* *C12N 15/55 (2006.01)*
*C12N 15/63 (2006.01)*

(52) Cooperative Patent Classification (CPC):
**A61K 31/7105; A61K 48/00; C12N 5/10; C12N 9/78; C12N 15/09; C12N 15/11; C12N 15/63**

(86) International application number:
**PCT/JP2020/029386**

(87) International publication number:
**WO 2021/020550 (04.02.2021 Gazette 2021/05)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **01.08.2019 JP 2019141875**

(71) Applicants:
- **Astellas Pharma Inc.**
**Chuo-ku**
**Tokyo 103-8411 (JP)**
- **Fukuoka University**
**Fukuoka-shi, Fukuoka 814-0180 (JP)**

(72) Inventors:
- **YOSHIMI Eiji**
**Tokyo 103-8411 (JP)**
- **MORIYA Yukari**
**Tokyo 103-8411 (JP)**
- **MANDA Mariko**
**Tokyo 103-8411 (JP)**
- **FUKUDA Masatora**
**Fukuoka-shi, Fukuoka 814-0180 (JP)**

(74) Representative: **Hoffmann Eitle**
**Patent- und Rechtsanwälte PartmbB**
**Arabellastraße 30**
**81925 München (DE)**

# (54) GUIDE RNA FOR TARGETED-EDITING WITH FUNCTIONAL BASE SEQUENCE ADDED THERETO

(57) [Problem to be Solved] To provide a guide RNA.
[Solution] A guide RNA for editing a target RNA sequence, comprising an antisense nucleotide sequence complementary to a portion of the target RNA sequence, a short-chain ADAR-recruiting nucleotide sequence, and at least one functional nucleotide sequence.

EP 4 008 784 A1

[Figure 1]

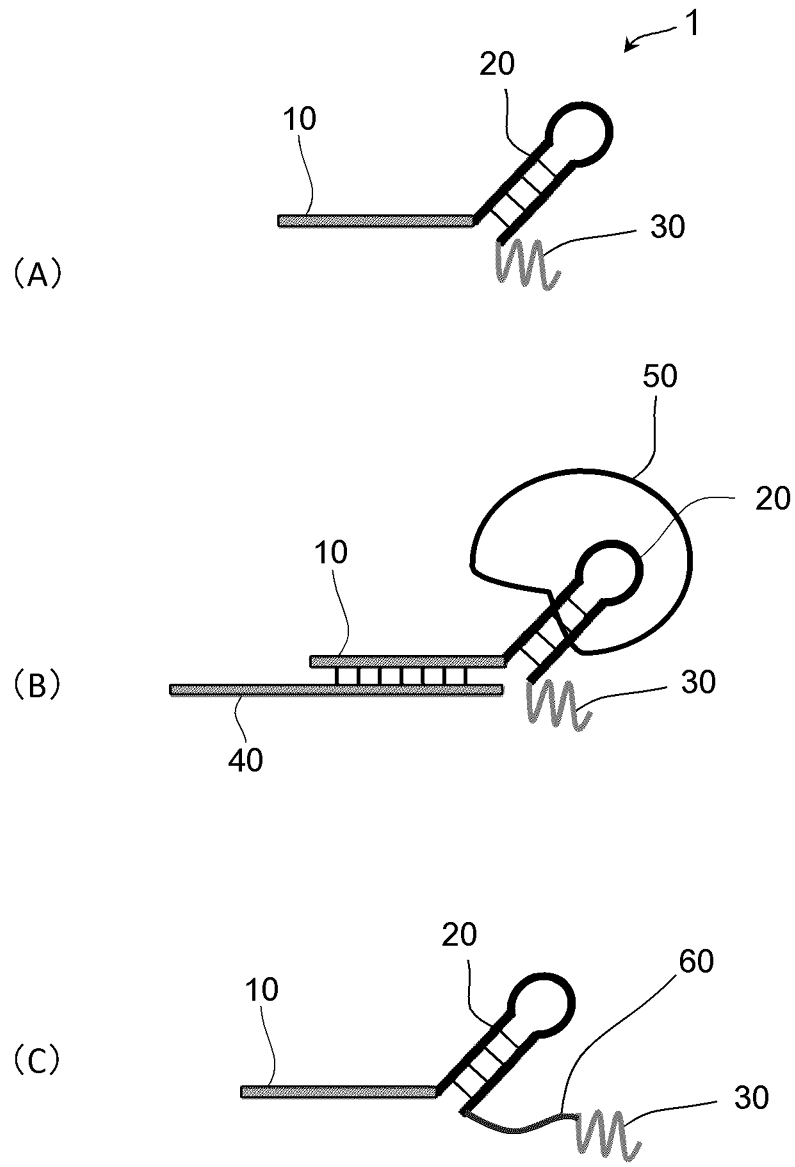

## Description

Technical Field

The present invention relates to a guide RNA for editing a target RNAby inducing ADAR, and in particular, to a guide RNA having a functional nucleotide sequence added thereto.

Background Art

An RNA-editing mechanism involving a single nucleotide mutation is present *in vivo.* In particular, RNA editing of converting adenosine (A) to inosine (I) by deamination of the adenosine (A) is most frequently observed, and the number of targets reaches 4,000,000. ADAR (Adenosine deaminases acting on RNA) has been known as an enzyme of editing adenosine to inosine, using a double-stranded RNA as a substrate. ADAR has three different gene subtypes (ADAR1, ADAR2, and ADAR3), and all of these subtypes have a double-stranded RNA-binding domain at the N-terminus thereof and a deaminase domain at the C-terminus thereof.

ADAR2 edits with high efficiency, subunit 2 (GluR2) that constitutes a 3-amino-3-hydroxy-5-methyl-4-isoxazole propionic acid (AMPA)-type glutamic acid receptor, in particular, in the central nervous system (Nature, 1996, Vol. 379, pp. 460-464 (Non Patent Literature 1)).

Since inosine converted from adenosine has a structure similar to guanosine, the inosine is recognized as guanosine at the stage of translation, and it is substituted for an amino acid in a coding region. It has been reported that a retrotransposon as a non-coding region (FEBS Letters, 2006, Vol. 580, pp. 2301-2305) or a microRNA (miRNA) precursor becomes a substrate of ADAR, and it has been suggested that ADAR be likely to play various functions *in vivo* (Ann. Rev. Biochem., 2010, Vol. 79, pp. 321-349).

Modified ADAR has also been reported. For example, it has been reported that a mutation is inserted into ADAR, so that the modified ADAR is capable of recognizing not only adenosine but also cytidine, and as a result, RNA editing of converting C to U, in which cytidine is converted to uridine by deamination of the cytidine, can be performed (Science, 2019, Vol. 365, pp. 382-386).

In recent years, RNA editing using a wild-type ADAR and an artificial guide RNA has been reported.

For example, as an RNA editing technique using, as a guide RNA, an antisense oligonucleotide that is an artificial synthetic nucleic acid, there has been known and reported a technique of using a single-stranded RNA having a length of 35 or more nucleotides complementary to an mRNA containing adenosine as an editing target, and forming a double strand having an incomplete helix structure with the mRNA, so as to recruit ADAR (International Publication WO2017/220751, International Publication WO2018/041973, and International Publication WO2018/134301). Moreover, a target RNA-editing guide RNA consisting of an oligonucleotide construct of two regions, namely, a targeting region comprising an antisense sequence complementary to a portion of a target RNA and GluR2-derived ADAR-recruiting region, has been reported (Patent Literature 1). Furthermore, it has been reported that a guide RNA designed based on the sequence of GluR2 was allowed to express in a wild-type ADAR2-expressing cell line, so that certain RNA editing was induced (Nucleic Acids Research, 2017, Vol. 45, pp. 2797-2808 (Non Patent Literature 2), and Patent Literatures 2 and 3), and that an endogenous target was edited using a guide RNA constituted with a modified oligonucleotide (Nat. Biotechnol., 2019, Vol. 37, pp. 133-138 (Non Patent Literature 3)).

In addition, a site-specific target RNA-editing guide RNA, comprising an antisense region binding to a target RNA and an ADAR-recruiting region, wherein the ADAR-recruiting region has a length of 49 nucleotides or 40 nucleotides, has been reported (Scientific Reports, 2017, Vol. 7, p. 41478 (Non Patent Literature 4), and Patent Literature 4). Recently, a target-editing guide RNA comprising a first oligonucleotide specifying a target RNA and a second oligonucleotide having a length of 2 to 34 nucleotides that is connected with the 3'-terminal side of the first oligonucleotide has been reported (Patent Literature 5). According to Patent Literature 5, if the number of residues of the second oligonucleotide is 14 or more, it is considered that a stable stem-loop structure is formed, and that editing activity is maintained.

There are several reports regarding addition of a nucleotide sequence to an RNA such as a guide RNA.

For instance, it has been known that the stem-loop structure that is the higher-order structure of a nucleic acid found in small RNA contributes to a translational regulation function (Cell. Mol. LifeSci., 2006, Vol. 63, pp. 901-908). ABoxB sequence derived from λ phage has a stem-loop structure and exhibits a strong affinity with a λN protein (Biol. Cell., 2008, Vol. 100, pp. 125-138, J. Am. Chem. Soc., 2002, Vol. 124, pp. 10966-10967). It has been reported that, in order to allow a guide RNA to recruit ADAR by utilizing the aforementioned properties of the BoxB sequence, the BoxB sequence is added to the guide RNA, and the λN protein is fused with ADAR (Nucleic Acids Research, 2016, Vol. 44, p.e157 (Non Patent Literature 6)). Patent Literature 2 discloses that the BoxB sequence can be added to the 3'-terminus of a guide RNA in order to stabilize the guide RNA designed based on the sequence of GluR2. Meanwhile, Non Patent Literature 2 discloses that the stabilization effect obtained by addition of the BoxB sequence to the guide RNA was low.

A G-quadruplex (Gq) structure is a repeating sequence comprising guanine, which is known as a DNA or RNA higher-order structure that is thermodynamically stable in an *in vivo* environment. Monovalent cations are necessary for formation of a higher-order structure. Gq has been originally reported as a sequence found in a telomere (Cell, 1989, Vol. 59, pp. 871-880). Known Gq functions are transcription, translation, epigenome regulation, and the like (EMBO Reports, 2015, Vol. 16, pp. 910-922). It has been reported that the efficiency of genome editing is improved by adding Gq to the 3'-terminus of the guide RNA of Cas9 (Chem. Commun., 2018, Vol. 54, pp. 2377-2380 (Non Patent Literature 5)).

U6 small nuclear (sn) RNA is a small RNA that is stably expressed in a large amount in all human cells, and the snRNA forms the ribonucleoprotein U6 snRNP in the nucleus. U6 snRNP constitutes a spliceosome and regulates splicing in an RNA precursor. A transcription start element located upstream of U6 is known as a strong polIII-type promoter for expressing any given RNA (ribozyme, antisense ribonucleic acid, RNA aptamer, etc.) (Gene Ther., 1997, Vol. 4, pp. 45-54). It has been reported that, in the case of a small RNA expression cassette formed with a sequence encoded by U6, the higher-order structure of a U6 sequence functions to locate an RNA inserted into the expression cassette in the nucleus (Gene Ther., 1997, Vol. 4, pp. 45-54 (Non Patent Literature 7), Nat. Biotechnol., 2002, Vol. 20, pp. 505-508 (Non Patent Literature 8), and Mol. Ther., 2003, Vol. 7, pp. 237-247 (Non Patent Literature 9)).

In view of the foregoing, there are several reports regarding RNA editing using an artificial guide RNA. In order to use a target RNA-editing guide RNA as a pharmaceutical product, it is necessary for such a guide RNA to show certain editing efficiency in cells. However, the improvement of the editing efficiency of a guide RNA has not been sufficiently considered yet.

## Citation List

### Patent Literature

Patent Literature 1: International Publication WO2016/097212
Patent Literature 2: International Publication WO2017/050306
Patent Literature 3: German Patent No. 102015012522
Patent Literature 4: International Publication WO2017/010556
Patent Literature 5: International Publication WO2019/111957

### Non Patent Literature

Non Patent Literature 1: Nature, 1996, Vol. 379, pp. 460-464
Non Patent Literature 2: Nucleic Acids Research, 2017, Vol. 45, pp. 2797-2808
Non Patent Literature 3: Nat. Biotechnol., 2019, Vol. 37, pp. 133-138
Non Patent Literature 4: Scientific Reports, 2017, Vol. 7, pp. 41478
Non Patent Literature 5: Chem. Commun., 2018, Vol. 54, pp. 2377-2380
Non Patent Literature 6: Nucleic Acids Research, 2016, Vol. 44, p. e157
Non Patent Literature 7: Gene Ther., 1997, Vol. 4, pp. 45-54
Non Patent Literature 8: Nat. Biotechnol., 2002, Vol. 20, pp. 505-508
Non Patent Literature 9: Mol. Ther., 2003, Vol. 7, pp. 237-247

## Summary of Invention

### Technical Problem

Provided are a guide RNA for editing a target RNA by inducing ADAR, and a nucleic acid encoding the same.

### Solution to Problem

The present inventors have conducted intensive studies regarding a guide RNA for editing a target RNA by inducing ADAR. As a result, the present inventors have found that a guide RNA having a functional nucleotide sequence added thereto shows high editing efficiency in cells, thereby completing the present invention.

Specifically, the present invention relates to the following [1] to [31].

[1] A guide RNA for editing a target RNA sequence, comprising:

an antisense nucleotide sequence complementary to a portion of the target RNA sequence, a short-chain ADAR-recruiting nucleotide sequence, and at least one functional nucleotide sequence.

[2] The guide RNA according to the above [1], wherein the antisense nucleotide sequence, the short-chain ADAR-recruiting nucleotide sequence, and the at least one functional nucleotide sequence are connected with one another, in this order, from the 5'-terminal side to the 3'-terminal side.

[3] The guide RNA according to the above [2], wherein at least one functional nucleotide sequence is further connected with the 5'-terminal side of the antisense nucleotide sequence.

[4] The guide RNA according to the above [1], further comprising a linker nucleotide sequence.

[5] The guide RNA according to the above [4], wherein at least one functional nucleotide sequence is connected with the short-chain ADAR-recruiting nucleotide sequence via the linker nucleotide sequence.

[6] The guide RNA according to the above [5], wherein the antisense nucleotide sequence, the short-chain ADAR-recruiting nucleotide sequence, the linker nucleotide sequence, and the at least one functional nucleotide sequence are connected with one another, in this order, from the 5'-terminal side to the 3'-terminal side.

[7] The guide RNA according to the above [6], wherein at least one functional nucleotide sequence is further connected with the 5'-terminal side of the antisense nucleotide sequence.

[8] The guide RNA according to any one of the above [1] to [7], wherein the functional nucleotide sequence is a nucleotide sequence associated with the intracellular stabilization and/or intracellular localization of the guide RNA.

[9] The guide RNA according to any one of the above [1] to [8], wherein the functional nucleotide sequence(s) are one or more nucleotide sequences selected from a nucleotide sequence that forms a G-quadruplex structure and a nucleotide sequence that forms a stem-loop structure.

[10] The guide RNA according to any one of the above [1] to [8], wherein the functional nucleotide sequence is a U6 snRNA sequence.

[11] The guide RNA according to the above [9], wherein the functional nucleotide sequence is a nucleotide sequence that forms a 1 to 4 layered G-quadruplex structure.

[12] The guide RNA according to the above [9], wherein the functional nucleotide sequence is a nucleotide sequence that forms a stem-loop structure.

[13] The guide RNA according to the above [12], wherein the nucleotide sequence that forms a stem-loop structure is a nucleotide sequence derived from a BoxB sequence.

[14] The guide RNA according to any one of the above [1] to [13], wherein the functional nucleotide sequence consists of the nucleotide sequence as set forth in SEQ ID NO: 6, 7, 8, or 9, or a nucleotide sequence comprising a deletion, substitution, insertion and/or addition of 1 to 3 nucleotides with respect to the nucleotide sequence as set forth in SEQ ID NO: 6, 7, 8, or 9, and having functions.

[15] The guide RNA according to any one of the above [1] to [14], wherein the short-chain ADAR-recruiting nucleotide sequence is a nucleotide sequence consisting of 14 to 34 nucleotides.

[16] The guide RNA according to the above [15], wherein the short-chain ADAR-recruiting nucleotide sequence is a sequence consisting of 16 nucleotides.

[17] The guide RNA according to the above [15] or [16], wherein the loop portion in the stem-loop structure of the short-chain ADAR-recruiting nucleotide sequence is a nucleotide sequence consisting of 4 or 5 nucleotides.

[18] The guide RNA according to any one of the above [15] to [17], wherein the stem portion in the stem-loop structure of the short-chain ADAR-recruiting nucleotide sequence has nucleotides that form a mismatched base pair.

[19] The guide RNA according to any one of the above [1] to [18], wherein the short-chain ADAR-recruiting nucleotide sequence consists of the nucleotide sequence as set forth in SEQ ID NO: 1, 2, 3, or 4, or a nucleotide sequence comprising a deletion, substitution, insertion and/or addition of 1 to 3 nucleotides with respect to the nucleotide sequence as set forth in SEQ ID NO: 1, 2, 3, or 4, and forming a stem-loop structure.

[20] The guide RNA according to any one of the above [4] to [19], wherein the linker nucleotide sequence is a nucleotide sequence consisting of 15 to 25 nucleotides.

[21] A target RNA sequence-editing system, comprising the guide RNA according to any one of the above [1] to [20] and ADAR

[22] A nucleic acid encoding the guide RNA according to any one of the above [1] to [20].

[23] An expression vector, comprising a nucleic acid encoding the guide RNA according to any one of the above [1] to [20].

[24] The expression vector according to the above [23], further comprising a nucleic acid encoding ADAR

[25] A host cell, into which the expression vector according to the above [23] or [24] is introduced.

[26] A method for producing an expression vector, comprising a step of culturing the host cell according to the above [25].

[27] A pharmaceutical composition, comprising the expression vector according to the above [23] or [24] and a pharmaceutically acceptable excipient.

[28] A pharmaceutical composition, comprising the expression vector according to the above [23], an expression vector containing a nucleic acid encoding ADAR, and a pharmaceutically acceptable excipient.

[29] The pharmaceutical composition according to the above [27] or [28], wherein the ADAR is human ADAR1 or human ADAR2

[30] The pharmaceutical composition according to the above [28] or [29], wherein the nucleic acid encoding ADAR is a nucleic acid consisting of a nucleotide sequence encoding the amino acid sequence as set forth in SEQ ID NO: 12, or a nucleotide sequence encoding a polypeptide that consists of an amino acid sequence having an identity of 90% or more to the amino acid sequence as set forth in SEQ ID NO: 12 and has an adenosine deaminase activity.

[31] A method for editing a target RNA, comprising: (i) a step of introducing the guide RNA according to any one of the above [1] to [20] or the nucleic acid according to the above [22] into a cell; (ii) a step of recruiting ADAR by the guide RNA; (iii) a step of forming a complex of a target RNA sequence and the guide RNA; (iv) a step of converting the adenosine of the target RNA sequence to inosine by the ADAR recruited by the guide RNA.

## Advantageous Effects of Invention

A guide RNA having a functional nucleotide sequence added thereto can be used as a guide RNA for editing a target RNA in a cell.

## Brief Description of Drawings

[Figure 1] Figure 1 is a schematic view showing the guide RNA of the present invention.

[Figure 2] Figure 2 shows representative examples of the two times experiments of luciferase assays (RNA-editing activity 3 days after the transfection). The longitudinal axis indicates a relative value of the Rluc/Fluc value of each guide RNA, when the Rluc/Fluc value of a guide RNA used as a control is set to be 1. The horizontal axis indicates the numbers (#) of plasmids. Besides, the experiments were each carried out for every 3 wells. The graph is shown as an arithmetic mean of the 3 wells and standard deviation.

[Figure 3] Figure 3 shows representative examples of the two times experiments of luciferase assays (RNA-editing activity 6 days after the transfection). The longitudinal axis indicates a relative value of the Rluc/Fluc value of each guide RNA, when the Rluc/Fluc value of a guide RNA used as a control is set to be 1. The horizontal axis indicates the numbers (#) of plasmids. Besides, the experiments were each carried out for every 3 wells. The graph is shown as an arithmetic mean of the 3 wells and standard deviation.

[Figure 4] Figure 4 shows the Rluc/Fluc value (RNA-editing activity 3 days after the transfection) obtained by luciferase assay. The longitudinal axis indicates the Rluc/Fluc value. The horizontal axis indicates the numbers (#) of plasmids. Besides, the experiment was carried out three times. The graph is shown as an arithmetic mean of the 3 trials and standard deviation. The plot indicates an arithmetic mean of every 3 samples in each trial.

## Description of Embodiments

The present invention will be described in detail below. However, the present invention is not limited to the following explanation. The terms used in the present description have meanings generally accepted by those skilled in the art, unless otherwise specifically defined.

### < Outline >

The present invention relates to a guide RNA for editing a target RNA sequence. According to RNA editing, for example, a specific nucleotide in a target sequence is substituted with another nucleotide, so that the function or expression of a protein encoded by the target sequence can be regulated (for example, can be enhanced or decreased).

Figure 1 is a schematic view of the guide RNA (1) of the present invention, and as shown in Figure 1A, the basic structure of the guide RNA (1) is composed of an antisense nucleotide sequence (10) complementary to a portion of a target RNA sequence, a short-chain ADAR-recruiting nucleotide sequence (20), and at least one functional nucleotide sequence (30).

The antisense nucleotide sequence (10) complementary to a portion of the target RNA sequence has a sequence complementary to the sequence of a partial region of the target RNA sequence. Thus, as shown in Figure 1B, the above-described antisense nucleotide sequence (10) can hybridize to a target RNA sequence (40). The short-chain ADAR-recruiting nucleotide sequence (20) is a nucleotide sequence that induces (recruits) an ADAR (50) and forms a complex with the ADAR (50). According to the thus recruited ADAR, a nucleotide in the target RNA sequence can be substituted with another nucleotide. The at least one functional nucleotide sequence (30) imparts any function such as intracellular stabilization or intracellular localization to the guide RNA.

As shown in Figure 1C, in the present invention, a linker (60) is further added to the above-described sequence, so that the guide RNA of the present invention can more effectively enhance the function of ADAR in cells.

Figure 1 is an illustrative example for showing a basic concept of the guide RNA of the present invention, and thus, the present invention is not limited to this aspect.

< Guide RNA of the present invention >

In the present invention, a guide RNA for editing a target RNA sequence (hereinafter also referred to as "the guide RNA of the present invention") means an RNA molecule that binds to ADAR and recruits the ADAR to a target RNA sequence. The guide RNA for editing a target RNA sequence of the present invention comprises an antisense nucleotide sequence complementary to a portion of the target RNA sequence, a short-chain ADAR-recruiting nucleotide sequence, and at least one functional nucleotide sequence. In a certain aspect, the guide RNA for editing a target RNA sequence of the present invention comprises an antisense nucleotide sequence complementary to a portion of the target RNA sequence, a short-chain ADAR-recruiting nucleotide sequence, a linker nucleotide sequence, and at least one functional nucleotide sequence.

In the present invention, the RNA may include an RNA comprising a modified nucleic acid and/or a nucleic acid analog. The modification that can be used in the present invention is, for example, nucleotide modification or skeletal modification, and such modification is well known to those skilled in the art.

Examples of a modified nucleic acid, which is used in the present invention, may include modified nucleotides such as 2'-O-alkyl ribose, 2'-O-methyl ribose, 2'-fluororibose, phosphorothioate, methylphosphonate, a phosphoramidite-morpholino oligomer (PMO), 5-methyl-dC, 2-amino-dA, and C5-pyrimidine. A preferred example of a stable modified nucleotide is a 2'-O-methyl modified nucleotide.

Examples of a nucleic acid analog used in the present invention may include a locked nucleic acid (LNA) nucleotide and/or a peptide nucleic acid (PNA).

In addition, in the present invention, the guide RNA may comprise a phosphorothioate bond between nucleotides.

1. Target RNA sequence

In the present invention, the "target RNA sequence" means an RNA sequence as a target of the editing according to the guide RNA of the present invention. In a certain aspect, the target RNA sequence is any given RNA sequence that comprises adenosine, wherein the adenosine is converted (edited) to inosine, so that the target RNA sequence can regulate cell function or gene expression. In another aspect, the target RNA sequence is any given RNA sequence that comprises cytidine, wherein the cytidine is converted (edited) to uridine, so that the target RNA sequence can regulate cell function or gene expression. The target RNA sequence can be present in an exon, an intron, a coding region in the exon, or various types of untranslated regions such as, for example, retrotransposon, or an RNA including a microRNA (miRNA), a transfer RNA (tRNA), and a ribosomal RNA (rRNA). The target RNA sequence can also be present in an RNA derived from virus.

2. Antisense nucleotide sequence

The guide RNA of the present invention comprises an antisense nucleotide sequence complementary to a portion of the target RNA sequence (which is simply referred to as an "antisense nucleotide sequence" at times). In the present invention, the "antisense nucleotide sequence" is a sequence that has a nucleotide sequence complementary to a portion of the target RNA sequence and forms a double strand with the target RNA sequence. The length of the antisense nucleotide sequence is, in a certain aspect, 10 nucleotides to 100 nucleotides, 10 nucleotides to 80 nucleotides, 10 nucleotides to 60 nucleotides, 10 nucleotides to 40 nucleotides, or 10 nucleotides to 30 nucleotides, in a certain aspect, 15 to 25 nucleotides, in a certain aspect, 18 to 25 nucleotides, in a certain aspect, 35 nucleotides to 40 nucleotides, and in a certain aspect, 70 nucleotides to 100 nucleotides. In a certain aspect, the antisense nucleotide sequence may comprise a nucleotide that forms a mismatched base pair with the target RNA sequence, or a nucleotide that forms a wobble base pair with the target RNA sequence.

The "mismatched base pairs" in the present description are the base pairs G-A, C-A, U-C, A-A, G-G, C-C, and U-U. The "wobble base pairs" in the present description are the base pairs G-U, I-U, I-A, and I-C.

The antisense nucleotide sequence is, in a certain aspect, a nucleotide sequence consisting of 18 to 25 nucleotides that has a nucleotide forming a mismatched base pair with adenosine of the target RNA and forms a complementary base pair with the target RNA.

The antisense nucleotide sequence can be designed, as appropriate, by those skilled in the art, while taking into consideration the sequence of the target RNA, the base length thereof, the position of a mismatched base pair formed with adenosine or cytidine of the target RNA, off-target effect, etc.

3. Short-chain ADAR-recruiting nucleotide sequence

In the present invention, the "ADAR-recruiting nucleotide sequence" is a nucleotide sequence that recruits ADAR and forms a stem-loop structure. The "short-chain ADAR-recruiting nucleotide sequence" is a short ADAR-recruiting nucleotide sequence, compared with an ADAR-recruiting nucleotide sequence consisting of 40 nucleotides (ARR(40), SEQ ID NO: 5) produced from an ADAR-recruiting nucleotide sequence consisting of 49 nucleotides derived from GluR2 (Patent Literature 4). The length of the short-chain ADAR-recruiting nucleotide sequence is, for example, 14 to 34 nucleotides, in a certain aspect, 14, 16, 24, or 34 nucleotides, in a certain aspect, 14 or 16 nucleotides, and in another aspect, 16 nucleotides.

In the present description, the phrase "to recruit ADAR" means that the nucleotide sequence binds to ADAR and induces the ADAR to the target RNA. The short-chain ADAR-recruiting nucleotide sequence of the present invention is not particularly limited, as long as it forms a structure that binds to ADAR. The stem-loop structure is also referred to as a hairpin structure, and it is publicly known in the present technical field. As known in the present technical field, since the stem-loop structure does not need the formation of a completely complementary base pair, the stem structure portion may comprise one or more mismatched base pairs or wobble base pairs. In a certain aspect, the short-chain ADAR-recruiting nucleotide sequence is a nucleotide sequence having a nucleotide that forms a mismatched base pair or a wobble base pair in a double-stranded RNA portion that forms a stem, and forming a stem-loop structure.

The short-chain ADAR-recruiting nucleotide sequence can be designed based on, for example, the stem-loop structure of the mRNA precursor of GluR2 (Patent Literature 4 and Non Patent Literature 4). In a certain aspect, the short-chain ADAR-recruiting nucleotide sequence can also be produced by shortening the stem portion of a nucleotide sequence forming the stem-loop structure of the mRNA precursor of GluR2, wherein the nucleotide sequence recruits ADAR, while retaining the recruiting function thereof.

In a certain aspect, the short-chain ADAR-recruiting nucleotide sequence may be a nucleotide sequence, in which the nucleotide sequence of a loop portion in the aforementioned stem portion-shortened nucleotide sequence is replaced with another nucleotide sequence that forms a loop. In a certain aspect, the short-chain ADAR-recruiting nucleotide sequence may be derived from an ADAR substrate other than the mRNA precursor of GluR2. Such an ADAR substrate that can be used in the designing of the ADAR-recruiting nucleotide sequence is well known to those skilled in the art (Biochemistry, 2018, Vol. 57, pp. 1640-1651, RNA, 2011, Vol. 2, pp. 761-771).

The nucleotide sequence of the loop structure portion of the short-chain ADAR-recruiting nucleotide sequence is not particularly limited, as long as it is a sequence that stably maintains a loop structure, from the viewpoint of nucleic acid chemistry. In a certain aspect, the nucleotide sequence of the loop structure portion is a nucleotide sequence consisting of GCUMA that is systematically conserved in GluR2 (wherein M represents A or C), and in a certain aspect, it is a nucleotide sequence consisting of GCUAA (Biophysical Chemistry, 2013, Vol. 180-181, pp. 110-118). In a certain aspect, the nucleotide sequence of the loop structure portion may be a nucleotide sequence that forms a tetraloop fold, and in another aspect, it is UUCG (ACS Chemical Biology, 2006, Vol. 1, pp. 761-765, Biophysical J., 2017, Vol. 113, pp. 257-267). The length of the loop structure portion is, in a certain aspect, 5 nucleotides, and it is, in a certain aspect, 4 nucleotides.

In a certain aspect, the short-chain ADAR-recruiting nucleotide sequence consists of the nucleotide sequence as set forth in SEQ ID NO: 1 (ARR(14)), or a nucleotide sequence having an identity of 90% or more to the nucleotide sequence as set forth in SEQ ID NO: 1, and forming a stem-loop structure, or a nucleotide sequence comprising a deletion, substitution, insertion and/or addition of 1 or 2 nucleotides with respect to the nucleotide sequence as set forth in SEQ ID NO: 1, and forming a stem-loop structure. In a certain aspect, the short-chain ADAR-recruiting nucleotide sequence consists of the nucleotide sequence as set forth in SEQ ID NO: 2 (ARR(16)), or a nucleotide sequence having an identity of 90% or more to the nucleotide sequence as set forth in SEQ ID NO: 2, and forming a stem-loop structure, or a nucleotide sequence comprising a deletion, substitution, insertion and/or addition of 1 or 2 nucleotides with respect to the nucleotide sequence as set forth in SEQ ID NO: 2, and forming a stem-loop structure. In a certain aspect, the short-chain ADAR-recruiting nucleotide sequence consists of the nucleotide sequence as set forth in SEQ ID NO: 3 (ARR(24)), or a nucleotide sequence having an identity of 90% or more to the nucleotide sequence as set forth in SEQ ID NO: 3, and forming a stem-loop structure, or a nucleotide sequence comprising a deletion, substitution, insertion and/or addition of 1 to 3 nucleotides with respect to the nucleotide sequence as set forth in SEQ ID NO: 3, and forming a stem-loop structure. In a certain aspect, the short-chain ADAR-recruiting nucleotide sequence consists of the nucleotide sequence as set forth in SEQ ID NO: 4 (ARR(34)), or a nucleotide sequence having an identity of 90% or more to the nucleotide sequence as set forth in SEQ ID NO: 4, and forming a stem-loop structure, or a nucleotide sequence comprising a deletion, substitution, insertion and/or addition of 1 to 4 nucleotides with respect to the nucleotide sequence as set forth in SEQ ID NO: 4, and forming a stem-loop structure.

In the present description, the term "ARR" means an ADAR-recruiting nucleotide sequence (ADAR Recruiting Region). The number in the parentheses after ARR indicates the base length of the ARR, and for example, "ARR(14)" means an ADAR-recruiting nucleotide sequence having a length of 14 nucleotides. The term "ASR" means an antisense nucleotide sequence (Antisense Region).

In the present description, the term "identity" means Identity that is a value obtained using parameters set to default values according to the EMBOSS NEEDLE program (J. Mol. Biol., 1970, Vol. 48, pp. 443-453) searching. The above-described parameters are as follows.

$$\text{Gap Open penalty} = 10$$

$$\text{Gap Extend penalty} = 0.5$$

$$\text{Matrix} = \text{EBLOSUM62}$$

4. Linker nucleotide sequence

The guide RNA of the present invention may comprise a linker nucleotide sequence. For example, by allowing the present guide RNA to comprise such a linker nucleotide sequence, the function or efficiency of the after-mentioned functional nucleotide sequence can be improved.

The linker nucleotide sequence means a nucleotide sequence that connects a certain nucleotide sequence with another nucleotide sequence, and the linker nucleotide sequence may be, for example, a nucleotide sequence that connects the short-chain ADAR-recruiting nucleotide sequence with a functional nucleotide sequence. In a certain aspect, the guide RNA of the present invention comprises one or more linker nucleotide sequences selected from among a linker nucleotide sequence that connects the short-chain ADAR-recruiting nucleotide sequence with a functional nucleotide sequence, a linker nucleotide sequence that connects the functional nucleotide sequence with the antisense nucleotide sequence, and a linker nucleotide sequence that connects a plurality of functional nucleotide sequences with one another.

The linker nucleotide sequence is, in a certain aspect, a nucleotide sequence that does not form a base pair with the target RNA sequence. The linker nucleotide sequence is, in a certain aspect, a nucleotide sequence that does not interact with the short-chain ADAR-recruiting nucleotide sequence. The linker nucleotide sequence is, in a certain aspect, a nucleotide sequence that neither forms a base pair with the target RNA sequence, nor interacts with the short-chain ADAR-recruiting nucleotide sequence. The linker nucleotide sequence is, in a certain aspect, a nucleotide sequence that forms a stem-loop structure in the linker nucleotide sequence itself. The linker nucleotide sequence is, in a certain aspect, a nucleotide sequence having moderate heat stability. The linker nucleotide sequence is, in a certain aspect, a nucleotide sequence that can immobilize the antisense nucleotide sequence and the short-chain ADAR-recruiting nucleotide sequence, to such an extent that ADAR can be stably induced to the target RNA sequence. The linker nucleotide sequence can be designed, as appropriate, by those skilled in the art, based on the sequence that constitutes the guide RNA. For example, the linker nucleotide sequence used in the present invention may be designed such that it does not form a complementary strand with the target RNA sequence. Otherwise, it is also possible to design the linker nucleotide sequence, such that a small loop is formed with 4 or 5 nucleotides.

The length of such a linker nucleotide sequence is, in a certain aspect, 15 to 25 nucleotides, and it is, in a certain aspect, 20 nucleotides. In a certain aspect, the linker nucleotide sequence consists of the nucleotide sequence as set forth in SEQ ID NO: 10 (add(20)), or a nucleotide sequence having an identity of 90% or more to the nucleotide sequence as set forth in SEQ ID NO: 10, or a nucleotide sequence comprising a deletion, substitution, insertion and/or addition of 1 or 2 nucleotides with respect to the nucleotide sequence as set forth in SEQ ID NO: 10.

In the present description, the term "add" means a linker nucleotide sequence.

In a certain aspect, the linker nucleotide sequence is a short linker nucleotide sequence (14 or less nucleotides). In a certain aspect, the guide RNA of the present invention may comprise a short linker nucleotide sequence, between the functional nucleotide sequence and the antisense nucleotide sequence, between the short-chain ADAR-recruiting nucleotide sequence and the functional nucleotide sequence, and/or between two or more of the functional nucleotide sequences. In a certain aspect, the guide RNA of the present invention may comprise a linker nucleotide sequence between the short-chain ADAR-recruiting nucleotide sequence and the functional nucleotide sequence, and may further comprise another short linker nucleotide sequence between the functional nucleotide sequence and the antisense nucleotide sequence, between the aforementioned linker nucleotide sequence and the functional nucleotide sequence, and/or between two or more of the functional nucleotide sequences. In a certain aspect, such a short linker nucleotide sequence consists of a nucleotide sequence that does not form a stem-loop structure in each single molecule. In a certain aspect, such a short linker nucleotide sequence consists of a nucleotide sequence that does not form a complementary strand with the target RNA. In a certain aspect, the length of such a short linker nucleotide sequence is 1 to 10 nucleotides, and it is 1 to 5 nucleotides in a certain aspect, 1 to 3 nucleotides in a certain aspect, 3 to 5 nucleotides in a certain aspect, and 3 nucleotides in a certain aspect. In a certain aspect, the short linker nucleotide sequence is "UCU." The short linker nucleotide sequence can be designed, as appropriate, by those skilled in the art, based on the sequence that constitutes the guide RNA. For example, the short linker nucleotide sequence used in the present invention may be designed, such that it does not form a complementary strand with the target RNA and it does not form a stem-loop structure in each single molecule of the linker.

5. Functional nucleotide sequence

The guide RNA of the present invention comprises at least one functional nucleotide sequence. In a certain aspect, the guide RNA of the present invention may comprise at least two functional nucleotide sequences of the same species. In another aspect, the guide RNA of the present invention may comprise at least two functional nucleotide sequences of two or more different species.

In the present invention, the "functional nucleotide sequence" means a nucleotide sequence that can impart to the guide RNA, any function such as the intracellular stabilization and/or intracellular localization of the guide RNA. Examples of the functional nucleotide sequence may include a nucleotide sequence that promotes the intracellular stabilization of the guide RNA, a nucleotide sequence that promotes localization of the guide RNA into the nucleus, and a nucleotide sequence including both of the two functions. In a certain aspect, the guide RNA of the present invention comprises, as such a functional nucleotide sequence, a nucleotide sequence that promotes the intracellular stabilization of the guide RNA, or a nucleotide sequence that promotes localization of the guide RNA into the nucleus, or both of the two nucleotide sequences. In a certain aspect, the nucleotide sequence that promotes the intracellular stabilization of the guide RNA includes a nucleotide sequence that forms a thermodynamically stabilizing higher-order structure. In a certain aspect, the nucleotide sequence that promotes localization of the guide RNA into the nucleus includes a nucleotide sequence that is derived from a small RNA (low molecular RNA) and promotes localization of the guide RNA into the nucleus. In a certain aspect, the functional nucleotide sequence includes a nucleotide sequence that forms a thermodynamically stabilizing higher-order structure and/or a nucleotide sequence that is derived from a small RNA and promotes localization of the guide RNA into the nucleus.

In a certain aspect, the guide RNA of the present invention comprises, as a functional nucleotide sequence(s), a nucleotide sequence that forms a thermodynamically stabilizing higher-order structure and/or a nucleotide sequence derived from a small RNA. The thermodynamically stabilizing higher-order structure is not particularly limited, as long as it is a structure that suppresses the decomposition of the guide RNA by nuclease. Examples of the thermodynamically stabilizing higher-order structure may include a double strand, a triple strand, a quadruple strand, and a stem-loop structure. In a certain aspect, examples of the functional nucleotide sequence may include a nucleotide sequence that forms a G-quadruplex (Gq) structure (Gq sequence) and/or a nucleotide sequence that forms a stem-loop structure. In a certain aspect, the guide RNA of the present invention comprises one or more functional nucleotide sequences selected from a Gq sequence and a nucleotide sequence that forms a stem-loop structure. The common structure of the Gq sequence is well known to those skilled in the art. The stem-loop structure is also referred to as a "hairpin structure," and is publicly known in the present technical field. Examples of the nucleotide sequence that forms a stem-loop structure may include an aptamer sequence (Int. J. Biochem. Mol. Biol., 2013, Vol. 4, pp. 27-40), a nucleotide sequence derived from a BoxB sequence, a nucleotide sequence derived from an MS2 sequence, and a nucleotide sequence derived from a PP7 sequence (Integr. Biol., 2009, Vol. 1, pp. 499-505, Nucleic Acids Research, 2016, Vol. 44, pp. 9555-9564). In a certain aspect, the guide RNA of the present invention comprises, as a functional nucleotide sequence, a nucleotide sequence derived from a BoxB sequence. In a certain aspect, the nucleotide sequence derived from a small RNA includes a U6 small nuclear (sn) RNA sequence.

The functional nucleotide sequence used in the present invention is, in a certain aspect, a Gq sequence, a U6 snRNA sequence, a BoxB sequence-derived nucleotide sequence, an MS2-derived nucleotide sequence, a PP7-derived nucleotide sequence, or a combination thereof. In a certain aspect, the functional nucleotide sequence used in the present invention is a Gq sequence, a U6 snRNA sequence, and/or a BoxB sequence-derived nucleotide sequence, or a combination thereof.

The Gq sequence used in the present invention is a sequence comprising 4 repeating units consisting of contiguous guanine residues, and 4 guanine residues form a planar quadruple strand (Gq) structure. The repeating units consisting of guanine residues may comprise nucleotides other than guanine among the units, as long as they maintain the Gq structure. In a certain aspect, the Gq sequence comprises repeating units each consisting of 1, 2, 3, or 4 guanine residues, and the repeating units each form 1, 2, 3, or 4 layers of Gq. In a certain aspect, the Gq sequence comprises repeating units each consisting of 3 guanine residues, and the repeating units form 3 layers of Gq. This is referred to as a "3 layered G-quadruplex structure" (which is also referred to as "3Gq" in the present description). In a certain aspect, the guide RNA of the present invention comprises 3Gq as a functional nucleotide sequence, and in a certain aspect, the nucleotide sequence that forms 3Gq consists of the nucleotide sequence as set forth in SEQ ID NO: 6, or a nucleotide sequence comprising a deletion, substitution, insertion and/or addition of 1 to 3 nucleotides with respect to the nucleotide sequence as set forth in SEQ ID NO: 6, and forming a Gq structure.

The U6 snRNA sequence used in the present invention is a sequence that is constituted with the nucleotide sequence of U6 snRNA. In the guide RNA of the present invention, the U6 snRNA sequence may be connected with the short-chain ADAR-recruiting nucleotide sequence and/or the antisense nucleotide sequence and may be then used, as long as it has the function of promoting localization of the guide RNA into the nucleus. Otherwise, the short-chain ADAR-recruiting nucleotide sequence and the antisense nucleotide sequence may be inserted into the U6 snRNA sequence, and may be then used. When the antisense nucleotide sequence and the short-chain ADAR-recruiting nucleotide sequence may be inserted into the U6 snRNA sequence and may be then used, the U6 snRNA sequence is divided into any given two regions (wherein the 5'-terminal side is referred to as an "upstream region", whereas the 3'-terminal side is referred to as a "downstream region"), and thereafter, the antisense nucleotide sequence and the short-chain ADAR-recruiting nucleotide sequence are inserted between the two regions and are then used. In a certain aspect of the guide RNA of the present invention comprising the U6 snRNA sequence, the antisense nucleotide sequence and the ADAR-recruiting nucleotide sequence are located between the upstream region of the U6 snRNA sequence and the downstream region of the U6 snRNA sequence, and thus, the upstream region of the U6 snRNA sequence, the antisense nucleotide sequence and the ADAR-recruiting nucleotide sequence, and the downstream region of the U6 snRNA sequence are connected with one another, in this order, from the 5'-terminal side to the 3'-terminal side. In a certain aspect, the nucleotide sequence of the upstream region of the U6 snRNA sequence used in the present invention consists of the nucleotide sequence as set forth in SEQ ID NO: 8 (also referred to as "U6 upstream"), or a nucleotide sequence comprising a deletion, substitution, insertion and/or addition of 1 to 3 nucleotides with respect to the nucleotide sequence as set forth in SEQ ID NO: 8, and having the function of promoting localization of the guide RNA into the nucleus when the nucleotide sequence is used together with the sequence of the downstream region of the U6 snRNA sequence. In a certain aspect, the nucleotide sequence of the downstream region of the U6 snRNA sequence used in the present invention consists of the nucleotide sequence as set forth in SEQ ID NO: 9 (also referred to as "U6 downstream"), or a nucleotide sequence comprising a deletion, substitution, insertion and/or addition of 1 to 3 nucleotides with respect to the nucleotide sequence as set forth in SEQ ID NO: 9, and having the function of promoting localization of the guide RNA into the nucleus when the nucleotide sequence is used together with the sequence of the upstream region of the U6 snRNA sequence.

In a certain aspect, the BoxB sequence-derived sequence used in the present invention consists of the nucleotide sequence as set forth in SEQ ID NO: 7 (which is also referred to as "BoxB"), or a nucleotide sequence having an identity of 90% or more to the nucleotide sequence as set forth in SEQ ID NO: 7, and forming a stem-loop structure, or a nucleotide sequence comprising a deletion, substitution, insertion and/or addition of 1 to 3 nucleotides with respect to the nucleotide sequence as set forth in SEQ ID NO: 7, and forming a stem-loop structure.

With regard to the functional nucleotide sequence, what the functional nucleotide sequence forms a Gq structure, forms a stem-loop structure, and has the function of promoting localization of the guide RNA into the nucleus when it is used together with the sequence of the upstream region or downstream region of the U6 snRNA sequence, is collectively referred to as "having functions," at times.

The functions of the functional nucleotide sequence to the guide RNA can be confirmed by known methods. Stabilization of the guide RNA can be confirmed, for example, by the methods described in Reference Examples 1 and 2, although the methods are not particularly limited thereto. It is considered that such stabilization of the guide RNA can reduce the dose of a pharmaceutical composition comprising the guide RNA, a nucleic acid encoding the guide RNA, and an expression vector, to a low dose.

6. Guide RNA for editing target RNA sequence

The guide RNA for editing a target RNA sequence of the present invention is a guide RNA comprising an antisense nucleotide sequence complementary to a portion of the target RNA sequence, a short-chain ADAR-recruiting nucleotide sequence, and at least one functional nucleotide sequence. Individual sequences that constitute the guide RNA may be directly connected with one another, or may be indirectly connected with one another via linker nucleotide sequences.

In a certain aspect, the short-chain ADAR-recruiting nucleotide sequence and the at least one functional nucleotide sequence are connected with one another via a linker nucleotide sequence(s). In a certain aspect, the at least one functional nucleotide sequence and the antisense nucleotide sequence are connected with one another via a linker nucleotide sequence(s). In a certain aspect, two or more of the functional nucleotide sequences are connected with one another via a linker nucleotide sequence(s). In a certain aspect, the short-chain ADAR-recruiting nucleotide sequence and the at least one functional nucleotide sequence are connected with one another via a linker nucleotide sequence(s);

and further, the functional nucleotide sequence and the antisense nucleotide sequence, the linker nucleotide sequence and the functional nucleotide sequence, and/or two or more of the functional nucleotide sequences may each be connected with each other via other short linker nucleotide sequences.

In a certain aspect, the guide RNA for editing a target RNA sequence of the present invention is any of the following guide RNAs:

a guide RNA, in which an antisense nucleotide sequence complementary to a portion of the target RNA sequence, a short-chain ADAR-recruiting nucleotide sequence, and at least one functional nucleotide sequence are connected with one another, in this order, from the 5'-terminal side to the 3'-terminal side,
a guide RNA, in which an antisense nucleotide sequence complementary to a portion of the target RNA sequence, a short-chain ADAR-recruiting nucleotide sequence, and at least one functional nucleotide sequence are connected with one another, in this order, from the 5'-terminal side to the 3'-terminal side, and further, at least one functional nucleotide sequence is further connected with the 5'-terminal side of the antisense nucleotide sequence,
a guide RNA, comprising an antisense nucleotide sequence complementary to a portion of the target RNA sequence, a short-chain ADAR-recruiting nucleotide sequence, a linker nucleotide sequence, and at least one functional nucleotide sequence,
a guide RNA, comprising an antisense nucleotide sequence complementary to a portion of the target RNA sequence, a short-chain ADAR-recruiting nucleotide sequence, a linker nucleotide sequence, and at least one functional nucleotide sequence, wherein the at least one functional nucleotide sequence is connected with the short-chain ADAR-recruiting nucleotide sequence via the linker nucleotide sequence,
a guide RNA, comprising an antisense nucleotide sequence complementary to a portion of the target RNA sequence, a short-chain ADAR-recruiting nucleotide sequence, a linker nucleotide sequence, and at least one functional nucleotide sequence, wherein the antisense nucleotide sequence, the short-chain ADAR-recruiting nucleotide sequence, the linker nucleotide sequence, and the at least one functional nucleotide sequence are connected with one another, in this order, from the 5'-terminal side to the 3'-terminal side, or
a guide RNA, comprising an antisense nucleotide sequence complementary to a portion of the target RNA sequence, a short-chain ADAR-recruiting nucleotide sequence, a linker nucleotide sequence, and at least one functional nucleotide sequence, wherein the antisense nucleotide sequence, the short-chain ADAR-recruiting nucleotide sequence, the linker nucleotide sequence, and the at least one functional nucleotide sequence are connected with one another, in this order, from the 5'-terminal side to the 3'-terminal side, and further, at least one functional nucleotide sequence is connected with the 5'-terminal side of the antisense nucleotide sequence.

In a certain aspect, the guide RNA for editing a target RNA sequence of the present invention is any of the following guide RNAs:

a guide RNA, comprising an antisense nucleotide sequence complementary to a portion of the target RNA sequence, a short-chain ADAR-recruiting nucleotide sequence, and at least one nucleotide sequence associated with the intracellular stabilization and/or intracellular localization of the guide RNA,
a guide RNA, comprising an antisense nucleotide sequence complementary to a portion of the target RNA sequence, a short-chain ADAR-recruiting nucleotide sequence, and at least one of a nucleotide sequence that forms a G-quadruplex structure and/or a nucleotide sequence that forms a stem-loop structure,
a guide RNA, comprising an antisense nucleotide sequence complementary to a portion of the target RNA sequence, a short-chain ADAR-recruiting nucleotide sequence, and at least one U6 snRNA sequence,
a guide RNA, comprising an antisense nucleotide sequence complementary to a portion of the target RNA sequence, a short-chain ADAR-recruiting nucleotide sequence, and at least one nucleotide sequence that forms a 1 to 4 layered G-quadruplex structure,
a guide RNA, comprising an antisense nucleotide sequence complementary to a portion of the target RNA sequence, a short-chain ADAR-recruiting nucleotide sequence, and at least one nucleotide sequence that forms a stem-loop structure,
a guide RNA, comprising an antisense nucleotide sequence complementary to a portion of the target RNA sequence, a short-chain ADAR-recruiting nucleotide sequence, and at least one BoxB sequence-derived nucleotide sequence, or
a guide RNA, comprising an antisense nucleotide sequence complementary to a portion of the target RNA sequence, a short-chain ADAR-recruiting nucleotide sequence, and at least one functional nucleotide sequence consisting of the nucleotide sequence as set forth in SEQ ID NO: 6, 7, 8, or 9, or a nucleotide sequence comprising a deletion, substitution, insertion and/or addition of 1 to 3 nucleotides with respect to the nucleotide sequence as set forth in SEQ ID NO: 6, 7, 8, or 9, and having functions.

In a certain aspect, the guide RNA for editing a target RNA sequence of the present invention is any of the following guide RNAs:

a guide RNA, comprising an antisense nucleotide sequence complementary to a portion of the target RNA sequence, a short-chain ADAR-recruiting nucleotide sequence consisting of 14 to 34 nucleotides, and at least one functional nucleotide sequence,
a guide RNA, comprising an antisense nucleotide sequence complementary to a portion of the target RNA sequence, a short-chain ADAR-recruiting nucleotide sequence consisting of 16 nucleotides, and at least one functional nucleotide sequence,
a guide RNA, comprising an antisense nucleotide sequence complementary to a portion of the target RNA sequence, a short-chain ADAR-recruiting nucleotide sequence consisting of 14 to 34 nucleotides, which comprises a loop portion consisting of 4 or 5 nucleotides, and at least one functional nucleotide sequence,
a guide RNA, comprising an antisense nucleotide sequence complementary to a portion of the target RNA sequence, a short-chain ADAR-recruiting nucleotide sequence consisting of 16 nucleotides, which comprises a loop portion consisting of 4 or 5 nucleotides, and at least one functional nucleotide sequence,
a guide RNA, comprising an antisense nucleotide sequence complementary to a portion of the target RNA sequence, a short-chain ADAR-recruiting nucleotide sequence consisting of 14 to 34 nucleotides, wherein a stem portion has a nucleotide that forms a mismatched base pair, and at least one functional nucleotide sequence,
a guide RNA, comprising an antisense nucleotide sequence complementary to a portion of the target RNA sequence, a short-chain ADAR-recruiting nucleotide sequence consisting of 16 nucleotides, wherein a stem portion has a nucleotide that forms a mismatched base pair, and at least one functional nucleotide sequence,
a guide RNA, comprising an antisense nucleotide sequence complementary to a portion of the target RNA sequence, a short-chain ADAR-recruiting nucleotide sequence consisting of 14 to 34 nucleotides, wherein a loop portion consists of 4 or 5 nucleotides and a stem portion has a nucleotide that forms a mismatched base pair, and at least one functional nucleotide sequence, or
a guide RNA, comprising an antisense nucleotide sequence complementary to a portion of the target RNA sequence, a short-chain ADAR-recruiting nucleotide sequence consisting of 16 nucleotides, wherein a loop portion consists of 4 or 5 nucleotides and a stem portion has a nucleotide that forms a mismatched base pair, and at least one functional nucleotide sequence.

In a certain aspect, the guide RNA for editing a target RNA sequence of the present invention is a guide RNA comprising an antisense nucleotide sequence complementary to a portion of the target RNA sequence, a short-chain ADAR-recruiting nucleotide sequence consisting of the nucleotide sequence as set forth in SEQ ID NO: 1, 2, 3, or 4, or a nucleotide sequence comprising a deletion, substitution, insertion and/or addition of 1 to 3 nucleotides with respect to the nucleotide sequence as set forth in SEQ ID NO: 1, 2, 3, or 4, and forming a stem-loop structure, and at least one functional nucleotide sequence.

In a certain aspect, the guide RNA for editing a target RNA sequence of the present invention is a guide RNA comprising an antisense nucleotide sequence complementary to a portion of the target RNA sequence, a short-chain ADAR-recruiting nucleotide sequence, a linker nucleotide sequence consisting of 15 to 25 nucleotides, and at least one functional nucleotide sequence. In a certain aspect, the guide RNA for editing a target RNA sequence of the present invention is a guide RNA comprising an antisense nucleotide sequence complementary to a portion of the target RNA sequence, a short-chain ADAR-recruiting nucleotide sequence, a linker nucleotide sequence consisting of 15 to 25 nucleotides and optionally having a stem-loop structure, and at least one functional nucleotide sequence.

In a certain aspect, the guide RNA for editing a target RNA sequence of the present invention is a guide RNA comprising:

an antisense nucleotide sequence complementary to a portion of the target RNA sequence,
a short-chain ADAR-recruiting nucleotide sequence, consisting of the nucleotide sequence as set forth in SEQ ID NO: 1 (ARR14), SEQ ID NO: 2 (ARR16), SEQ ID NO: 3 (ARR24), or SEQ ID NO: 4 (ARR34), or a nucleotide sequence comprising a deletion, substitution, insertion and/or addition of 1 to 3 nucleotides with respect to the nucleotide sequence as set forth in SEQ ID NO: 1, 2, 3, or 4, and forming a stem-loop structure, and
at least one functional nucleotide sequence, consisting of the nucleotide sequence as set forth in SEQ ID NO: 6 (3Gq), SEQ ID NO: 7 (BoxB), or SEQ ID NO: 8 (U6 upstream) and SEQ ID NO: 9 (U6 downstream), or a nucleotide sequence comprising a deletion, substitution, insertion and/or addition of 1 to 3 nucleotides with respect to any nucleotide sequence as set forth in SEQ ID NO: 6, 7, 8, or 9, and having functions.

In a certain aspect, the guide RNA for editing a target RNA sequence of the present invention is a guide RNA comprising:

an antisense nucleotide sequence complementary to a portion of the target RNA sequence,
a short-chain ADAR-recruiting nucleotide sequence, consisting of the nucleotide sequence as set forth in SEQ ID NO: 1 (ARR14), SEQ ID NO: 2 (ARR16), SEQ ID NO: 3 (ARR24), or SEQ ID NO: 4 (ARR34), or a nucleotide sequence comprising a deletion, substitution, insertion and/or addition of 1 to 3 nucleotides with respect to the nucleotide sequence as set forth in SEQ ID NO: 1, 2, 3, or 4, and forming a stem-loop structure,
a linker nucleotide sequence, consisting of the nucleotide sequence as set forth in SEQ ID NO: 10 (add(20)), or a nucleotide sequence comprising a deletion, substitution, insertion and/or addition of 1 to 3 nucleotides with respect to the nucleotide sequence as set forth in SEQ ID NO: 10, and forming a stem-loop structure, and
at least one functional nucleotide sequence, consisting of the nucleotide sequence as set forth in SEQ ID NO: 6 (3Gq), SEQ ID NO: 7 (BoxB), or SEQ ID NO: 8 (U6 upstream) and SEQ ID NO: 9 (U6 downstream), or a nucleotide sequence comprising a deletion, substitution, insertion and/or addition of 1 to 3 nucleotides with respect to any nucleotide sequence as set forth in SEQ ID NO: 6, 7, 8, or 9, and having functions nucleotide sequence.

In a certain aspect, the guide RNA for editing a target RNA sequence of the present invention is a guide RNA comprising an antisense nucleotide sequence complementary to a portion of the target RNA sequence, a short-chain ADAR-recruiting nucleotide sequence, and at least one functional nucleotide sequence, and the present guide RNA has any of the following characteristics:

(1) the antisense nucleotide sequence complementary to a portion of the target RNA sequence that is connected with the 5'-terminal side of the short-chain ADAR-recruiting nucleotide sequence,
(2) the antisense nucleotide sequence complementary to a portion of the target RNA sequence that is connected with the 5'-terminal side of the short-chain ADAR-recruiting nucleotide sequence, and the functional nucleotide sequence that is connected with the 3'-terminal side of the short-chain ADAR-recruiting nucleotide sequence,
(3) the antisense nucleotide sequence complementary to a portion of the target RNA sequence that is connected with the 5'-terminal side of the short-chain ADAR-recruiting nucleotide sequence, and the functional nucleotide sequence that is connected with the 5'-terminal side of the antisense nucleotide sequence, or
(4) the antisense nucleotide sequence complementary to a portion of the target RNA sequence that is connected with the 5'-terminal side of the short-chain ADAR-recruiting nucleotide sequence, and the functional nucleotide sequence that is connected with both of the 5'-terminal side of the antisense nucleotide sequence and the 3'-terminal side of the short-chain ADAR-recruiting nucleotide sequence.

The guide RNA of the present invention having any of the above-described (1) to (4) may comprise a linker nucleotide sequence. In the guide RNA of the present invention, the antisense nucleotide sequence, the short-chain ADAR-recruiting nucleotide sequence, and the functional nucleotide sequence(s) may be directly connected with one another, or the antisense nucleotide sequence, the short-chain ADAR-recruiting nucleotide sequence, and the functional nucleotide sequence(s) may also be connected with one another via the linker nucleotide sequence. In a certain aspect, the short-chain ADAR-recruiting nucleotide sequence and the functional nucleotide sequence are connected with each other via the linker nucleotide sequence.

In the guide RNA of the present invention having any of the above-described characteristics (1) to (4), the short-chain ADAR-recruiting nucleotide sequence is, in a certain aspect, an ADAR-recruiting nucleotide sequence consisting of 14 to 34 nucleotides, or in a certain aspect, an ADAR-recruiting nucleotide sequence consisting of 16 nucleotides. In a certain aspect, the short-chain ADAR-recruiting nucleotide sequence consists of the nucleotide sequence as set forth in SEQ ID NO: 1, 2, 3, or 4, or a nucleotide sequence comprising a deletion, substitution, insertion and/or addition of 1 to 3 nucleotides with respect to the nucleotide sequence as set forth in SEQ ID NO: 1, 2, 3, or 4, and forming a stem-loop structure.

In a certain aspect, the guide RNA of the present invention having any of the above-described characteristics (1) to (4) comprises at least one functional nucleotide sequence selected from among a Gq sequence, a BoxB sequence-derived sequence, and a U6 snRNA sequence. In a certain aspect, the guide RNA of the present invention having any of the above-described characteristics (1) to (4) comprises, as a functional nucleotide sequence(s), at least one functional nucleotide sequence selected from among: a Gq sequence consisting of the nucleotide sequence as set forth in SEQ ID NO: 6, or a nucleotide sequence comprising a deletion, substitution, insertion and/or addition of 1 to 3 nucleotides with respect to the nucleotide sequence as set forth in SEQ ID NO: 6, and forming a Gq structure; a BoxB sequence-derived sequence consisting of the nucleotide sequence as set forth in SEQ ID NO: 7, or a nucleotide sequence comprising a deletion, substitution, insertion and/or addition of 1 to 3 nucleotides with respect to the nucleotide sequence as set forth in SEQ ID NO: 7, and forming a stem-loop structure; or a U6 snRNA sequence consisting of a combination of the nucleotide sequence as set forth in SEQ ID NO: 8, or a nucleotide sequence comprising a deletion, substitution, insertion and/or addition of 1 to 3 nucleotides with respect to the nucleotide sequence as set forth in SEQ ID NO: 8, and having the function of promoting localization of the guide RNA into the nucleus when the nucleotide sequence is used together with the sequence of the downstream region of the U6 snRNA sequence, and the nucleotide sequence as set forth in SEQ ID NO: 9, or a nucleotide sequence comprising a deletion, substitution, insertion and/or addition of 1 to 3 nucleotides with respect to the nucleotide sequence as set forth in SEQ ID NO: 9, and having the function of promoting localization of the guide RNA into the nucleus when the nucleotide sequence is used together with the sequence of the upstream region of the U6 snRNA sequence.

< Method for producing guide RNA of the present invention >

The guide RNA of the present invention can be synthesized based on the sequence information according to a standard polynucleotide synthesis method that is known in the present technical field. In addition, if a certain guide RNA of the present invention is obtained, a mutation is introduced into a predetermined site of the certain guide RNA by applying a method known to those skilled in the art, such as site-directed mutagenesis (Current Protocols in Molecular Biology, 1987, John Wiley & Sons Section), so that another guide RNA of the present invention that maintains the function of inducing ADAR to the target RNA sequence and the function of editing the target RNA sequence can be produced. The guide RNA of the present invention can also be produced using a chemically modified nucleic acid.

< ADAR used in the present invention >

The ADAR used in the present invention includes naturally occurring ADAR, and a modified form thereof, as long as it has a deaminase activity. In a certain aspect, the ADAR used in the present invention is eukaryote-derived ADAR, and in another aspect, the present ADAR is mammal-derived ADAR. In a certain aspect, the ADAR used in the present invention is human ADAR, in a certain aspect, the present ADAR is ADAR1 or ADAR2, and in a certain aspect, it is ADAR2 The ADAR1 includes 2 types of splicing variants, ADAR1 p110 and ADAR1 p150. The ADAR used in the present invention is, in a certain aspect, ADAR1 p110 or ADAR1 p150. The ADAR used in the present invention is, in a certain aspect, human ADAR1 or human ADAR2, and it is, in a certain aspect, human ADAR2.

In a certain aspect, the ADAR used in the present invention is a polypeptide comprising a double-stranded RNA binding domain (dsRBD) and having a deaminase enzyme activity (Trends in Biochemical Sciences, 2001, Vol. 26, pp. 376-384, RNA, 2001, Vol. 7, pp. 846-858).

In a certain aspect, the ADAR used in the present invention is a polypeptide that comprises an adenosine deaminase domain and is recruited by the guide RNA of the present invention. In another aspect, the ADAR used in the present invention is a polypeptide that comprises a deaminase domain and can convert the cytidine of the target RNA to uridine.

The ADAR used in the present invention may be a fusion protein with another factor. Examples of a modified form of the ADAR used in the present invention may include a modified form having an adenosine deaminase activity, a modified form having a cytidine deaminase activity, and a modified form having a deaminase activity that recognizes both adenosine and cytidine and converts them to inosine and uridine, respectively. Such a modified form of the ADAR used in the present invention may be a fusion protein with another factor.

In a certain aspect, the ADAR used in the present invention is a polypeptide consisting of the amino acid sequence with Accession No. [NP_001103.1], Accession No. [NP_056648.1] or Accession No. [NP_001102.3], or a polypeptide consisting of an amino acid sequence having an identity of 90% or more to the aforementioned amino acid sequences, or an amino acid sequence comprising a deletion, substitution, insertion and/or addition of 1 to 10 amino acids with respect to the aforementioned amino acid sequences, and having an adenosine deaminase activity. In a certain aspect, the ADAR used in the present invention is a polypeptide consisting of the amino acid sequence as set forth in SEQ ID NO: 12 (human ADAR2), or a polypeptide consisting of an amino acid sequence having an identity of 90% or more to the amino acid sequence as set forth in SEQ ID NO: 12, or an amino acid sequence comprising a deletion, substitution, insertion and/or addition of 1 to 10 amino acids with respect to the amino acid sequence as set forth in SEQ ID NO: 12, and having an adenosine deaminase activity. In a certain aspect, the ADAR used in the present invention may be ADAR that is endogenously present in a eukaryotic cell, or in a certain aspect, the present ADAR may also be ADAR that is exogenously introduced into a eukaryotic cell. With regard to introduction of the ADAR into the eukaryotic cell, the ADAR polypeptide may be directly into the cell, or an expression vector containing a nucleic acid encoding the ADAR may be introduced into the cell.

< Target RNA sequence-editing system of the present invention >

The present invention also provides a system for editing a target RNA sequence, comprising the guide RNA of the present invention and ADAR. The system for editing a target RNA sequence, comprising the guide RNA of the present invention and ADAR, includes a kit for editing a target RNA sequence, comprising the guide RNA of the present invention and ADAR, or a method for editing a target RNA sequence, using the guide RNA of the present invention and ADAR. The target RNA sequence-editing system of the present invention is a system comprising: a guide RNA comprising an antisense nucleotide sequence complementary to a portion of the target RNA sequence, a short-chain ADAR-recruiting nucleotide sequence, and at least one functional nucleotide sequence; and ADAR. In a certain aspect, the target RNA sequence-editing system of the present invention is a system comprising: a guide RNA comprising an antisense nucleotide sequence complementary to a portion of the target RNA sequence, a short-chain ADAR-recruiting nucleotide sequence, and at least one functional nucleotide sequence; and human ADAR1 or human ADAR2 The target RNA sequence-editing system of the present invention can be used, either intracellularly or extracellularly. In a certain aspect, the present target RNA sequence-editing system can be used in a eukaryotic cell.

< Nucleic acid encoding guide RNA of the present invention >

The nucleic acid encoding the guide RNA of the present invention is a nucleic acid encoding a guide RNA comprising an antisense nucleotide sequence complementary to a portion of the target RNA sequence, a short-chain ADAR-recruiting nucleotide sequence, and at least one functional nucleotide sequence. Individual sequences comprised in the guide RNA may be directly connected with one another, or may also be connected with one another via a linker nucleotide sequence.

In a certain aspect, the nucleic acid encoding the guide RNA of the present invention is any of the following nucleic acids:

a nucleic acid encoding a guide RNA comprising an antisense nucleotide sequence complementary to a portion of the target RNA sequence, a short-chain ADAR-recruiting nucleotide sequence, and at least one functional nucleotide sequence,

a nucleic acid encoding a guide RNA, in which an antisense nucleotide sequence complementary to a portion of the target RNA sequence, a short-chain ADAR-recruiting nucleotide sequence, and at least one functional nucleotide sequence are connected with one another, in this order, from the 5'-terminal side to the 3'-terminal side,

a nucleic acid encoding a guide RNA, in which an antisense nucleotide sequence complementary to a portion of the target RNA sequence, a short-chain ADAR-recruiting nucleotide sequence, and at least one functional nucleotide sequence are connected with one another, in this order, from the 5'-terminal side to the 3'-terminal side, and further, at least one functional nucleotide sequence is further connected with the 5'-terminal side of the antisense nucleotide sequence,

a nucleic acid encoding a guide RNA comprising an antisense nucleotide sequence complementary to a portion of the target RNA sequence, a short-chain ADAR-recruiting nucleotide sequence, a linker nucleotide sequence, and at least one functional nucleotide sequence,

a nucleic acid encoding a guide RNA comprising an antisense nucleotide sequence complementary to a portion of the target RNA sequence, a short-chain ADAR-recruiting nucleotide sequence, a linker nucleotide sequence, and at least one functional nucleotide sequence, wherein the at least one functional nucleotide sequence is connected with the short-chain ADAR-recruiting nucleotide sequence via the linker nucleotide sequence,

a nucleic acid encoding a guide RNA comprising an antisense nucleotide sequence complementary to a portion of the target RNA sequence, a short-chain ADAR-recruiting nucleotide sequence, a linker nucleotide sequence, and at least one functional nucleotide sequence, wherein the antisense nucleotide sequence, the short-chain ADAR-recruiting nucleotide sequence, the linker nucleotide sequence, and the at least one functional nucleotide sequence are connected with one another, in this order, from the 5'-terminal side to the 3'-terminal side, or

a nucleic acid encoding a guide RNA comprising an antisense nucleotide sequence complementary to a portion of the target RNA sequence, a short-chain ADAR-recruiting nucleotide sequence, a linker nucleotide sequence, and at least one functional nucleotide sequence, wherein the antisense nucleotide sequence, the short-chain ADAR-recruiting nucleotide sequence, the linker nucleotide sequence, and the at least one functional nucleotide sequence are connected with one another, in this order, from the 5'-terminal side to the 3'-terminal side, and further, at least one functional nucleotide sequence is connected with the 5'-terminal side of the antisense nucleotide sequence.

In a certain aspect, the nucleic acid encoding the guide RNA of the present invention is any of the following nucleic acids:

a nucleic acid encoding a guide RNA comprising an antisense nucleotide sequence complementary to a portion of the target RNA sequence, a short-chain ADAR-recruiting nucleotide sequence, and at least one nucleotide sequence associated with the intracellular stabilization and/or intracellular localization of the guide RNA,

a nucleic acid encoding a guide RNA comprising an antisense nucleotide sequence complementary to a portion of the target RNA sequence, a short-chain ADAR-recruiting nucleotide sequence, and at least one of a nucleotide sequence that forms a G-quadruplex structure and/or a nucleotide sequence that forms a stem-loop structure,

a nucleic acid encoding a guide RNA comprising an antisense nucleotide sequence complementary to a portion of the target RNA sequence, a short-chain ADAR-recruiting nucleotide sequence, and at least one U6 snRNA sequence,
a nucleic acid encoding a guide RNA comprising an antisense nucleotide sequence complementary to a portion of the target RNA sequence, a short-chain ADAR-recruiting nucleotide sequence, and at least one nucleotide sequence that forms a 1 to 4 layered G-quadruplex structure,
a nucleic acid encoding a guide RNA comprising an antisense nucleotide sequence complementary to a portion of the target RNA sequence, a short-chain ADAR-recruiting nucleotide sequence, and at least one nucleotide sequence that forms a stem-loop structure,
a nucleic acid encoding a guide RNA comprising an antisense nucleotide sequence complementary to a portion of the target RNA sequence, a short-chain ADAR-recruiting nucleotide sequence, and at least one BoxB sequence-derived nucleotide sequence, or
a nucleic acid encoding a guide RNA comprising an antisense nucleotide sequence complementary to a portion of the target RNA sequence, a short-chain ADAR-recruiting nucleotide sequence, and at least one functional nucleotide sequence consisting of the nucleotide sequence as set forth in SEQ ID NO: 6, 7, 8, or 9, or a nucleotide sequence comprising a deletion, substitution, insertion and/or addition of 1 to 3 nucleotides with respect to the nucleotide sequence as set forth in SEQ ID NO: 6, 7, 8, or 9, and having functions.

In a certain aspect, the nucleic acid encoding the guide RNA of the present invention is a nucleic acid encoding any of the following guide RNAs:

a nucleic acid encoding a guide RNA comprising an antisense nucleotide sequence complementary to a portion of the target RNA sequence, a short-chain ADAR-recruiting nucleotide sequence consisting of 14 to 34 nucleotides, and at least one functional nucleotide sequence,
a nucleic acid encoding a guide RNA comprising an antisense nucleotide sequence complementary to a portion of the target RNA sequence, a short-chain ADAR-recruiting nucleotide sequence consisting of 16 nucleotides, and at least one functional nucleotide sequence,
a nucleic acid encoding a guide RNA comprising an antisense nucleotide sequence complementary to a portion of the target RNA sequence, a short-chain ADAR-recruiting nucleotide sequence consisting of 14 to 34 nucleotides, which comprises a loop portion consisting of 4 or 5 nucleotides, and at least one functional nucleotide sequence,
a nucleic acid encoding a guide RNA comprising an antisense nucleotide sequence complementary to a portion of the target RNA sequence, a short-chain ADAR-recruiting nucleotide sequence consisting of 16 nucleotides, which comprises a loop portion consisting of 4 or 5 nucleotides, and at least one functional nucleotide sequence,
a nucleic acid encoding a guide RNA comprising an antisense nucleotide sequence complementary to a portion of the target RNA sequence, a short-chain ADAR-recruiting nucleotide sequence consisting of 14 to 34 nucleotides, wherein a stem portion has a nucleotide that forms a mismatched base pair, and at least one functional nucleotide sequence,
a nucleic acid encoding a guide RNA comprising an antisense nucleotide sequence complementary to a portion of the target RNA sequence, a short-chain ADAR-recruiting nucleotide sequence consisting of 16 nucleotides, wherein a stem portion has a nucleotide that forms a mismatched base pair, and at least one functional nucleotide sequence,
a nucleic acid encoding a guide RNA comprising an antisense nucleotide sequence complementary to a portion of the target RNA sequence, a short-chain ADAR-recruiting nucleotide sequence consisting of 14 to 34 nucleotides, wherein a loop portion consists of 4 or 5 nucleotides and a stem portion has a nucleotide that forms a mismatched base pair, and at least one functional nucleotide sequence, or
a nucleic acid encoding a guide RNA comprising an antisense nucleotide sequence complementary to a portion of the target RNA sequence, a short-chain ADAR-recruiting nucleotide sequence consisting of 16 nucleotides, wherein a loop portion consists of 4 or 5 nucleotides and a stem portion has a nucleotide that forms a mismatched base pair, and at least one functional nucleotide sequence.

In a certain aspect, the nucleic acid encoding the guide RNA of the present invention is a nucleic acid encoding a guide RNA comprising an antisense nucleotide sequence complementary to a portion of the target RNA sequence, a short-chain ADAR-recruiting nucleotide sequence consisting of the nucleotide sequence as set forth in SEQ ID NO: 1, 2, 3, or 4, or a nucleotide sequence comprising a deletion, substitution, insertion and/or addition of 1 to 3 nucleotides with respect to the nucleotide sequence as set forth in SEQ ID NO: 1, 2, 3, or 4, and forming a stem-loop structure, and at least one functional nucleotide sequence.
In a certain aspect, the nucleic acid encoding the guide RNA of the present invention is a nucleic acid encoding a guide RNA comprising an antisense nucleotide sequence complementary to a portion of the target RNA sequence, a short-chain ADAR-recruiting nucleotide sequence, a linker nucleotide sequence consisting of 15 to 25 nucleotides and optionally having a stem-loop structure, and at least one functional nucleotide sequence.

In a certain aspect, the nucleic acid encoding the guide RNA of the present invention is a nucleic acid encoding a guide RNA comprising an antisense nucleotide sequence complementary to a portion of the target RNA sequence, a short-chain ADAR-recruiting nucleotide sequence consisting of the nucleotide sequence as set forth in SEQ ID NO: 1, 2, 3, or 4, or a nucleotide sequence comprising a deletion, substitution, insertion and/or addition of 1 to 3 nucleotides with respect to the nucleotide sequence as set forth in SEQ ID NO: 1, 2, 3, or 4, and forming a stem-loop structure, a linker nucleotide sequence that is the nucleotide sequence as set forth in SEQ ID NO: 10, or a nucleotide sequence comprising a deletion, substitution, insertion and/or addition of 1 to 3 nucleotides with respect to the nucleotide sequence as set forth in SEQ ID NO: 10, and at least one functional nucleotide sequence consisting of the nucleotide sequence as set forth in SEQ ID NO: 6, 7, 8, or 9, or a nucleotide sequence comprising a deletion, substitution, insertion and/or addition of 1 to 3 nucleotides with respect to the nucleotide sequence as set forth in SEQ ID NO: 6, 7, 8, or 9, and having functions.

In a certain aspect, the nucleic acid encoding the guide RNA of the present invention includes a nucleic acid encoding a guide RNA comprising an antisense nucleotide sequence complementary to a portion of the target RNA sequence, a short-chain ADAR-recruiting nucleotide sequence, a linker nucleotide sequence, and at least one functional nucleotide sequence, wherein nucleic acids encoding the short-chain ADAR-recruiting nucleotide sequence, the linker nucleotide sequence, and the at least one functional nucleotide sequence each consist of the following nucleotide sequences, or nucleotide sequences each comprising a deletion, substitution, insertion and/or addition of 1 to 3 nucleotides with respect to the following nucleotide sequences.

The nucleic acid sequence encoding the short-chain ADAR-recruiting nucleotide sequence:
the nucleic acid encoding ARR(16): SEQ ID NO: 13

The nucleic acid sequence encoding the linker nucleotide sequence:
the nucleic acid encoding add(20): SEQ ID NO: 14

The nucleic acids encoding the functional nucleotide sequences:

the nucleic acid encoding 3Gq: SEQ ID NO: 15
the nucleic acid encoding BoxB: SEQ ID NO: 16
the nucleic acid encoding U6 upstream: SEQ ID NO: 17
the nucleic acid encoding U6 downstream: SEQ ID NO: 18

The nucleic acid encoding the guide RNA of the present invention is, for example, a DNA or a modified DNA, and in a certain aspect, the nucleic acid encoding the guide RNA of the present invention is a DNA.

In a certain aspect, the nucleic acid encoding the guide RNA of the present invention is a nucleic acid that is incorporated into an expression vector. In a certain aspect, the nucleic acid encoding the guide RNA of the present invention is a nucleic acid that is incorporated into a plasmid vector. In a certain aspect, the nucleic acid encoding the guide RNA of the present invention is a nucleic acid that is incorporated into a viral vector.

< Nucleic acid encoding ADAR used in the present invention >

The nucleic acid encoding the ADAR used in the present invention is a nucleic acid encoding the ADAR described in the above < ADAR used in the present invention >. The nucleic acid encoding the ADAR used in the present invention is, in a certain aspect, a nucleic acid consisting of the nucleotide sequence encoding the amino acid sequence with Accession No. [NP_001103.1], Accession No. [NP_056648.1] or Accession No. [NP_001102.3], or a nucleic acid consisting of a nucleotide sequence encoding a polypeptide that consists of an amino acid sequence having an identity of 90% or more to at least one of these sequences or an amino acid sequence comprising a deletion, substitution, insertion and/or addition of 1 to 10 amino acids with respect to at least one of these sequences, and has an adenosine deaminase activity. The nucleic acid encoding the ADAR used in the present invention is, in a certain aspect, a nucleic acid consisting of the nucleotide sequence as set forth in SEQ ID NO: 11, or a nucleic acid encoding a polypeptide that consists of a nucleotide sequence having an identity of 90% or more to the nucleotide sequence as set forth in SEQ ID NO: 11, or a nucleotide sequence comprising a deletion, substitution, insertion and/or addition of 1 to 10 nucleotides with respect to the nucleotide sequence as set forth in SEQ ID NO: 11, and has an adenosine deaminase activity.

< Expression vector of the present invention >

The present invention also provides an expression vector containing the nucleic acid encoding the guide RNA of the present invention (which is also referred to as "the expression vector of the present invention"). The expression vector of the present invention may further comprise a nucleic acid encoding ADAR The nucleic acid encoding the guide RNA of the present invention and the nucleic acid encoding the ADAR used in the present invention may be incorporated into a single expression vector, or may also be incorporated into different expression vectors. In a certain aspect, the expression vector of the present invention is a combination of an expression vector containing the nucleic acid encoding the guide RNA of the present invention and an expression vector containing the nucleic acid encoding ADAR In a certain aspect, the expression vector of the present invention is an expression vector containing the nucleic acid encoding the guide RNA of the present invention and the nucleic acid encoding ADAR

1. Expression vector used in the present invention

The expression vector used in the present invention is not particularly limited, as long as it can express the guide RNA of the present invention from the nucleic acid encoding the guide RNA of the present invention, and/or can express the ADAR used in the present invention. In a certain aspect, the expression vector used in the present invention is an expression vector that can be used to express the guide RNA of the present invention and/or the ADAR used in the present invention in a human cell. In a certain aspect, examples of the expression vector used in the present invention may include plasmid vectors and viral vectors (e.g. an adenoviral vector, a retroviral vector, and an adeno-associated viral vector).

2. Promoter

The expression vector of the present invention may comprise a promoter that is operably linked to the nucleic acid encoding the guide RNA of the present invention and/or the nucleic acid encoding the ADAR used in the present invention. In the present description, the phrase "be operably linked" means that at least one promoter is linked to a nucleic acid, such that a polypeptide or an RNA encoded by the nucleic acid can be expressed in a host cell.

The promoter comprised in the expression vector of the present invention is not particularly limited. A promoter corresponding to RNA polymerase (for example, polII or polIII) suitable for the expression of the nucleic acid encoding the guide RNA of the present invention or the ADAR used in the present invention can be used. In order to allow the guide RNA of the present invention to express, in a certain aspect, a promoter corresponding to polII or polIII can be used, and in another aspect, a promoter corresponding to polIII can be used. In order to allow the ADAR used in the present invention to express, in a certain aspect, a promoter corresponding to polII can be used.

Examples of the promoter corresponding to polII may include a CMV (cytomegalovirus)-derived promoter, an SV40 (simian virus 40) promoter, an RSV (respiratory syncytial virus) promoter, an EF1$\alpha$ (Elongation factor 1$\alpha$) promoter, and a CAG promoter. Examples of the promoter corresponding to polIII may include a human U6 small nuclear RNA (snRNA) promoter (U6) (Nat. Biotechnol., 2002, Vol. 20, pp. 497-500), a highly sensitive U6 promoter (Nucleic Acids Research, 2003, Vol. 31, p.e100), a human HI promoter, and other viral and eukaryotic cell promoters known to those skilled in the art, but the examples are not limited thereto.

The expression vector of the present invention may further comprise a translation initiation codon, a translation termination codon, a purine base (G or A) preferable for the transcription start point of polIII, a polyA signal, a terminator sequence made of consecutive T residues for polIII, an enhancer, an untranslated region, a splicing junction, etc., depending on the type of a promoter or host cells used, etc.

3. Expression vector of the present invention

The expression vector of the present invention is an expression vector containing the nucleic acid encoding the guide RNA of the present invention. In a certain aspect, the expression vector of the present invention is a combination of an expression vector containing the nucleic acid encoding the guide RNA of the present invention and an expression vector containing the nucleic acid encoding ADAR In a certain aspect, the expression vector of the present invention is an expression vector containing the nucleic acid encoding the guide RNA of the present invention and the nucleic acid encoding ADAR

The expression vector of the present invention is an expression vector containing a nucleic acid encoding a guide RNA, wherein the guide RNA comprises an antisense nucleotide sequence complementary to a portion of the target RNA sequence, a short-chain ADAR-recruiting nucleotide sequence, and at least one functional nucleotide sequence.

In a certain aspect, the expression vector of the present invention is an expression vector containing a nucleic acid encoding a guide RNA, wherein the guide RNA comprises an antisense nucleotide sequence complementary to a portion of the target RNA sequence, a short-chain ADAR-recruiting nucleotide sequence, a linker nucleotide sequence, and at least one functional nucleotide sequence.

< Host cell of the present invention >

The present invention also provides a host cell, into which the nucleic acid encoding the guide RNA of the present invention is introduced (which is also referred to as "the host cell of the present invention"). In a certain aspect, the host cell of the present invention is a host cell, into which an expression vector containing the nucleic acid encoding the guide RNA of the present invention is introduced. In a certain aspect, the host cell of the present invention is a host cell, into which an expression vector containing the nucleic acid encoding the guide RNA of the present invention and the nucleic acid encoding ADAR is introduced. In a certain aspect, the host cell of the present invention is a host cell, into which an expression vector containing the nucleic acid encoding the guide RNA of the present invention and an expression vector containing the nucleic acid encoding ADAR are introduced. In a certain aspect, the host cell of the present invention is a host cell, into which the expression vector of the present invention that is a plasmid vector is introduced. In a certain aspect, the host cell of the present invention is a host cell, into which a plasmid vector for producing the expression vector of the present invention that is a viral vector is introduced.

The host cell, into which the expression vector of the present invention is introduced, is not particularly limited. A cell known in the present technical field can be selected, as long as it is a cell that can be used in replication of the nucleic acid encoding the guide RNA of the present invention. Examples of the host cell that can be used in replication of the vector may include various cells, such as natural cells commonly used in the technical field of the present invention or artificially established cells. Specific examples of the host cell that can be used in replication of the vector may include animal cells (e.g. CHO cells, HEK293 cells, etc.), insect cells (e.g. Sf9 cells, etc.), bacteria (*Escherichia coli*, etc.), and yeasts (genus Saccharomyces, genus Pichia, etc.). In a certain aspect, *Escherichia coli* can be used as a host cell. Introduction itself can be carried out according to a known method (Green, M.R. and Sambrook, J., Molecular Cloning: A Laboratory Manual, 4th Edition, Cold Spring Harbor Laboratory Press, 2012).

< Method for producing nucleic acid encoding guide RNA of the present invention and expression vector of the present invention >

The nucleic acid encoding the guide RNA of the present invention (hereinafter also referred to as "the nucleic acid of the present invention") can be synthesized based on the sequence described in the present description or publicly available sequence information, according to a standard polynucleotide synthesis method that is known in the present technical field. In addition, if a certain nucleic acid of the present invention is obtained, a mutation is introduced into a predetermined site of the certain nucleic acid by applying a method known to those skilled in the art, such as site-directed mutagenesis (Current Protocols in Molecular Biology, 1987, John Wiley & Sons Section), so that another nucleic acid of the present invention can be produced.

The present invention includes a method for producing a nucleic acid or an expression vector, comprising a step of culturing host cells, into which the nucleic acid of the present invention or an expression vector containing the nucleic acid of the present invention is introduced. In a certain aspect, the method for producing the nucleic acid of the present invention comprises a step of culturing host cells, into which the nucleic acid of the present invention is introduced, so that the nucleic acid of the present invention is replicated. In a certain aspect, the method for producing the nucleic acid and expression vector of the present invention comprises a step of culturing host cells, into which an expression vector containing the nucleic acid of the present invention is introduced, so that the expression vector of the present invention is replicated. When the expression vector of the present invention is a viral vector, the method for producing the expression vector of the present invention comprises a step of culturing host cells, into which a plasmid vector used in generation of a viral vector containing the nucleic acid of the present invention is introduced, or host cells, into which a viral vector containing the nucleic acid of the present invention is introduced, and then purifying a viral vector produced in the host cells. Such a viral vector can be produced according to a method known to those skilled in the art.

The method for producing the nucleic acid of the present invention and the expression vector of the present invention may include a step of recovering a culture solution of the host cells to obtain a lysate (lysis solution). Such a lysate can be obtained, for example, by treating the recovered culture solution according to an alkaline lysis method or a boiling method. The method for producing the nucleic acid and expression vector of the present invention may further comprise a step of purifying a nucleic acid or an expression vector from the lysate. For purification of a nucleic acid or an expression vector from the lysate, ion exchange chromatography and/or hydrophobic interaction chromatography can be used.

When the expression vector of the present invention is a viral vector, in order to purify a viral vector from the lysate, cesium chloride density-gradient centrifugation, sucrose gradient centrifugation, iodixanol density-gradient centrifugation, ultrafiltration, diafiltration, affinity chromatography, ion exchange chromatography, polyethylene glycol precipitation, ammonium sulfate precipitation, or the like can be used.

< Pharmaceutical composition of the present invention >

The present invention also provides a pharmaceutical composition comprising the guide RNA of the present invention, the nucleic acid of the present invention, or the expression vector of the present invention, and a pharmaceutically acceptable excipient (also referred to as "the pharmaceutical composition of the present invention").

In a certain aspect, the pharmaceutical composition of the present invention is a pharmaceutical composition comprising the guide RNA of the present invention and a pharmaceutically acceptable excipient. In a certain aspect, the pharmaceutical composition of the present invention is a pharmaceutical composition comprising the guide RNA of the present invention, ADAR, and a pharmaceutically acceptable excipient.

In a certain aspect, the pharmaceutical composition of the present invention is a pharmaceutical composition comprising the nucleic acid of the present invention and a pharmaceutically acceptable excipient. In a certain aspect, the pharmaceutical composition of the present invention is a pharmaceutical composition comprising the nucleic acid of the present invention, ADAR, and a pharmaceutically acceptable excipient.

In a certain aspect, the pharmaceutical composition of the present invention is a pharmaceutical composition comprising the expression vector of the present invention and a pharmaceutically acceptable excipient. In a certain aspect, the pharmaceutical composition of the present invention is a pharmaceutical composition comprising an expression vector containing the nucleic acid encoding the guide RNA of the present invention and the nucleic acid encoding ADAR, and a pharmaceutically acceptable excipient. In a certain aspect, the pharmaceutical composition of the present invention is a pharmaceutical composition comprising an expression vector containing the nucleic acid encoding the guide RNA of the present invention, a vector containing the nucleic acid encoding ADAR, and a pharmaceutically acceptable excipient.

In a certain aspect, the pharmaceutical composition of the present invention is a pharmaceutical composition comprising an expression vector containing the nucleic acid encoding the guide RNA of the present invention and the nucleic acid encoding human ADAR1 or human ADAR2, and a pharmaceutically acceptable excipient. In a certain aspect, the pharmaceutical composition of the present invention is a pharmaceutical composition comprising an expression vector containing the nucleic acid encoding the guide RNA of the present invention, a vector containing the nucleic acid encoding human ADAR1 or human ADAR2, and a pharmaceutically acceptable excipient. In a certain aspect, the pharmaceutical composition of the present invention is a pharmaceutical composition comprising an expression vector containing the nucleic acid encoding the guide RNA of the present invention and the nucleic acid encoding ADAR, wherein the nucleic acid consists of a nucleotide sequence encoding the amino acid sequence as set forth in SEQ ID NO: 12 or a nucleotide sequence encoding a polypeptide that consists of an amino acid sequence having an identity of 90% or more to the amino acid sequence as set forth in SEQ ID NO: 12 and has an adenosine deaminase activity, and a pharmaceutically acceptable excipient. In a certain aspect, the pharmaceutical composition of the present invention is a pharmaceutical composition comprising: an expression vector containing the nucleic acid encoding the guide RNA of the present invention; an expression vector containing the nucleic acid encoding ADAR, wherein the nucleic acid consists of a nucleotide sequence encoding the amino acid sequence as set forth in SEQ ID NO: 12 or a nucleotide sequence encoding a polypeptide that consists of an amino acid sequence having an identity of 90% or more to the amino acid sequence as set forth in SEQ ID NO: 12 and has an adenosine deaminase activity; and a pharmaceutically acceptable excipient.

The pharmaceutical composition of the present invention can be prepared using excipients commonly used in the present technical field, such as pharmaceutical excipients or pharmaceutical carriers, according to a commonly used method. Examples of the dosage form of such a pharmaceutical composition may include parenteral agents such as injections and intravenous drips, which can be administered via intravenous administration, subcutaneous administration, intracutaneous administration, intramuscular administration, etc. For formulation of the present pharmaceutical composition, excipients, carriers, additives and the like, which depend on the aforementioned dosage forms, can be used within a pharmaceutically acceptable range.

The applied dose of the guide RNA of the present invention, the nucleic acid of the present invention, an expression vector containing the nucleic acid encoding the guide RNA of the present invention, or an expression vector containing the nucleic acid encoding the guide RNA of the present invention and the nucleic acid encoding ADAR, is different depending on the degree of symptoms or age of a patient, the dosage form of a preparation used, etc. For example, it can be used at a dose of approximately 0.001 mg/kg to 100 mg/kg. Moreover, the guide RNA of the present invention, the nucleic acid of the present invention, an expression vector containing the nucleic acid encoding the guide RNA of the present invention, or an expression vector containing the nucleic acid encoding the guide RNA of the present invention and the nucleic acid encoding ADAR is added in an amount corresponding to the aforementioned applied dose, so as to formulation a preparation.

The applied dose of the expression vector containing the ADAR used in the present invention or the nucleic acid encoding the ADAR can be appropriately adjusted, depending on cells in which the target RNA is present, etc. The expression vector can be used, for example, at a dose of approximately 0.001 mg/kg to 100 mg/kg.

In a certain aspect, the disease, which can be prevented or treated with the guide RNA, the nucleic acid, or the expression vector of the present invention, is a hereditary disease, and in a certain aspect, it is any given disease, to which a modification of one or more adenosine or cytidine residues in the target RNA provides an advantageous change. The pharmaceutical composition of the present invention can be used as a preventive or therapeutic agent for any given disease, to which a modification of one or more adenosine or cytidine residues in the target RNA provides an advantageous change. In addition, the pharmaceutical composition of the present invention can be used as a preventive or therapeutic agent for any given disease, to which a modification of one or more adenosine residues in the target RNA provides an advantageous change.

The present invention includes a pharmaceutical composition for use in preventing or treating any given disease, to which a modification of one or more adenosine or cytidine residues in the target RNA provides an advantageous change, wherein the pharmaceutical composition comprises the guide RNA of the present invention, the nucleic acid of the present invention, or the expression vector of the present invention. In addition, the present invention includes a method for preventing or treating any given disease, to which a modification of one or more adenosine or cytidine residues in the target RNA provides an advantageous change, wherein the method comprises a step of administering to a patient, a preventively effective amount or therapeutically effective amount of the guide RNA of the present invention, the nucleic acid of the present invention, or the expression vector of the present invention. Moreover, the present invention includes the guide RNA of the present invention, the nucleic acid of the present invention, or the expression vector of the present invention, which is for use in preventing or treating any given disease, to which a modification of one or more adenosine or cytidine residues in the target RNA provides an advantageous change. Furthermore, the present invention includes use of the guide RNA of the present invention, the nucleic acid of the present invention, or the expression vector of the present invention, in the production of a pharmaceutical composition for preventing or treating any given disease, to which a modification of one or more adenosine or cytidine residues in the target RNA provides an advantageous change.

< Method for editing target RNA sequence, using guide RNA of the present invention >

The present invention also provides a method for editing a target RNA sequence, using the guide RNA of the present invention (also referred to as "the editing method of the present invention"). In a certain aspect, the editing method of the present invention includes a method by which a target RNA sequence is allowed to react with the guide RNA of the present invention to form a complex, and ADAR recruited by the guide RNA converts the adenosine of the target RNA sequence to inosine. Herein, the adenosine on the target RNA sequence that is to be converted to inosine may form a mismatched base pair with an antisense nucleotide sequence. In a certain aspect, the editing method of the present invention includes a method by which a target RNA sequence is allowed to react with the guide RNA of the present invention to form a complex, and ADAR recruited by the guide RNA converts the cytidine of the target RNA sequence to uridine. Herein, the cytidine on the target RNA sequence that is to be converted to uridine may form a mismatched base pair with an antisense nucleotide sequence.

The editing method comprises: (i) a step of introducing a guide RNA comprising an antisense nucleotide sequence complementary to a portion of the target RNA sequence, a short-chain ADAR-recruiting nucleotide sequence, and at least one functional nucleotide sequence, or a nucleic acid encoding the guide RNA, into cells, (ii) a step of recruiting ADAR by the guide RNA, (iii) a step of forming a complex of the target RNA sequence and the guide RNA; (iv) a step of converting the adenosine of the target RNA sequence to inosine by the ADAR recruited by the guide RNA. The editing method may comprise a step of introducing ADAR or a nucleic acid encoding the ADAR, and a guide RNA or a nucleic acid encoding the guide RNA, into cells, simultaneously or separately.

The editing method includes a method of converting the adenosine of a target sequence to inosine, or a method of converting the cytidine of a target sequence to uridine, in cells, or in eukaryotic cells according to a certain aspect, or in mammalian cells according to a certain aspect. Further, when such a target RNA sequence is present in a coding region, the editing method may comprise a step of reading inosine as guanosine upon translation.

## Examples

The steps described in the following Examples can be carried out according to known methods, unless otherwise particularly specified. In addition, in steps in which commercially available kits or reagents, etc. were used, experiments were carried out according to protocols included therewith, unless otherwise particularly specified.

### Example 1: Production of guide RNA expression plasmid comprising Gq sequence and/or BoxB sequence-derived sequence as functional nucleotide sequence(s)

A guide RNA expression plasmid comprising a Gq sequence and/or a BoxB sequence-derived sequence as functional nucleotide sequence(s) and also comprising ARR(16) as an ADAR-recruiting nucleotide sequence was produced as follows. Specifically, DNA sequences encoding 6 types of guide RNAs (please refer to the below-mentioned < Names of guide RNAs and sequence numbers of DNAs encoding guide RNAs >) were each inserted into a site located downstream of an HI RNA polymerase III promoter (hereinafter referred to as "H1 promoter") of a pSUPER.neo vector (Oligoengine, Catalog No. VEC-PBS-0004), using the restriction enzymes BglII (on the 5'-terminal side) and HindIII (on the 3'-terminal side). Upon production of the DNA sequences to be inserted, the DNA sequences were produced according to either DNA synthesis (FASMAC, Thermo Fisher Scientific), or a method of annealing a forward oligo and a reverse oligo (as described below) that were synthesized to form restriction enzyme ends.

Forward oligo: 5'-GATC-"DNA sequence encoding guide RNA excluding restriction enzyme recognition sequence"-3'

Reverse oligo: 5'-AGCT- "reverse complementary sequence of DNA sequence encoding guide RNA excluding restriction enzyme recognition sequence"-3'

The produced guide RNA expression plasmids are collectively referred to as "pSUPERneo_H1ADg."

< Names of guide RNAs and sequence numbers of DNAs encoding guide RNAs >

ASR(24)-ARR(16)add-3Gq: SEQ ID NO: 19
ASR(24)-ARR(16)add-BoxB: SEQ ID NO: 20
BoxB-ASR(24)-ARR(16)add-3Gq: SEQ ID NO: 21
ASR(24)-ARR(16)-3Gq: SEQ ID NO: 22
ASR(24)-ARR(16)-BoxB: SEQ ID NO: 23
BoxB-ASR(24)-ARR(16)-3Gq: SEQ ID NO: 24

The DNA sequence comprises a purine base (G or A) preferable for the transcription start point of polIII on the 5'-terminal side, and a terminator sequence of polIII on the 3'-terminal side. Moreover, the DNA sequence comprises the restriction enzyme BglII recognition sequence (AGATCT) and the restriction enzyme HindII recognition sequence (AAGCTT), which are used in vector cloning, at the 5'-terminus and at the 3'-terminus, respectively.

Herein, ASR(24) means an antisense nucleotide sequence consisting of 24 nucleotides complementary to a part of a reporter mRNA (Rluc-W104X) for detection of RNA editing used in Example 3, and the ASR(24) consists of an RNA sequence encoded by a DNA sequence from nucleotide numbers 10 to 33 as set forth in SEQ ID NO: 19. ARR(16) consists of the RNA sequence as set forth in SEQ ID NO: 2, and the nucleic acid encoding ARR(16) consists of the DNA sequence as set forth in SEQ ID NO: 13. Moreover, "add" consists of the RNA sequence as set forth in SEQ ID NO: 10, and the nucleic acid encoding the "add" consists of the DNA sequence as set forth in SEQ ID NO: 14. Furthermore, 3Gq consists of the RNA sequence as set forth in SEQ ID NO: 6, and the nucleic acid encoding the 3Gq consists of the DNA sequence as set forth in SEQ ID NO: 15. Further, BoxB consists of the RNA sequence as set forth in SEQ ID NO: 7, and the nucleic acid encoding the BoxB consists of the DNA sequence as set forth in SEQ ID NO: 16.

An outline of the DNA sequences encoding individual guide RNAs is shown in Table 1. In Table 1, the term "5'-" means the functional nucleotide sequence on the 5'-terminal side, and the "3'-" means the functional nucleotide sequence on the 3'-terminal side. The term "SEQ ID NO:" means the DNA sequence number encoding each guide RNA.

*E. coli* DH5α Competent Cells (Takara Bio, Inc., Catalog No. 9057; hereinafter referred to as an *"Escherichia coli* strain DH5α") or OneShot Stbl3 Chemically Competent *E. coli* (Thermo Fisher Scientific, Catalog No. C737303; hereinafter referred to as an *"Escherichia coli* strain Stbl3") were transformed with the plasmid pSUPERneo H1ADg, and were then subjected to a liquid culture. The culture solution was centrifuged, and the E. coliwas then collected. Thereafter, plasmid extraction and purification were carried out using NucleoBond(registered trademark) Xtra Midi Plus EF (Takara Bio, Inc., Catalog No. U0422B), so as to obtain the amplified plasmid pSUPERneo H1ADg.

Besides, the guide RNA expression plasmids used as controls or comparative examples were produced in the same manner as that described above, using the DNA sequences (as shown below) encoding the guide RNAs.

< Name of guide RNA and sequence number of DNA encoding guide RNA (control) >

ASR(24)-ARR(40): SEQ ID NO: 27

< Names of guide RNAs and sequence numbers of DNAs encoding guide RNAs (comparative examples) >

ASR(24)-ARR(16)add: SEQ ID NO: 28
ASR(24)-ARR(16): SEQ ID NO: 29
ASR(24)-ARR(40)add: SEQ ID NO: 30
ASR(24)-ARR(40)add-3Gq: SEQ ID NO: 31
ASR(24)-ARR(40)add-BoxB: SEQ ID NO: 32

BoxB-ASR(24)-ARR(40)add-3Gq: SEQ ID NO: 33
ASR(24)-ARR(40)-3Gq: SEQ ID NO: 35
ASR(24)-ARR(40)-BoxB: SEQ ID NO: 36
BoxB-ASR(24)-ARR(40)-3Gq: SEQ ID NO: 37

The DNA sequence comprises a purine base (G or A) preferable for the transcription start point of polIII on the 5'-terminal side, and a terminator sequence of polIII on the 3'-terminal side. Moreover, the DNA sequence comprises the restriction enzyme BglII recognition sequence (AGATCT) and the restriction enzyme HindII recognition sequence (AAGCTT), which are used in vector cloning, at the 5'-terminus and at the 3'-terminus, respectively.

An outline of the DNA sequences encoding individual guide RNAs is shown in Table 1.

Example 2: Production of guide RNA expression plasmid, to which U6 snRNA sequence is added

A vector was constructed by substituting the HI promoter sequence of a pSUPER.neo vector with a human U6 RNA polymerase III (hU6) promoter sequence (SEQ ID NO: 39) (hereinafter referred to as a "pSUPER.neo-U6 vector"). A fragment comprising a hU6 promoter sequence was amplified by using, as a template, pBAsi-hU6 Neo DNA (Takara Bio, Inc., Catalog No. 3227) having a hU6 promoter, and also using primers to which individual restriction enzyme sites were added (i.e. EcoRI on the 5'-terminal side and BglII on the 3'-terminal side), according to a standard PCR method. For the reaction, Tks Gflex DNA polymerase (Takara Bio, Inc., Catalog No. R060A) was used. The obtained hU6 promoter fragment was inserted into the pSUPER.neo vector, using the restriction enzymes EcoRI (on the 5'-terminal side) and BglII (on the 3'-terminal side). The constructed pSUPER.neo-U6 vector was amplified using the *Escherichia coli* strain DH5$\alpha$ or the *Escherichia coli* strain Stbl3.

A guide RNA expression plasmid, to which a U6 snRNA sequence was added, was constructed as follows. DNA sequences encoding two types of guide RNAs (as shown below) were inserted into a site located downstream of the hU6 promoter of the pSUPER.neo-U6 vector, using the restriction enzymes BglII (on the 5'-terminal side) and HindIII (on the 3'-terminal side). The DNA sequences to be inserted were produced by the same method as that of Example 1.

< Names of guide RNAs and sequence numbers of DNAs encoding guide RNAs >

U6 upstream-ASR(24)-ARR(16)add-U6 downstream: SEQ ID NO: 25
U6 upstream-ASR(24)-ARR(16)-U6 downstream: SEQ ID NO: 26

The DNA sequence comprises a purine base (G or A) preferable for the transcription start point of polIII on the 5'-terminal side, and a terminator sequence of polIII on the 3'-terminal side. Moreover, the DNA sequence comprises the restriction enzyme BglII recognition sequence (AGATCT) and the restriction enzyme HindII recognition sequence (AAGCTT), which are used in vector cloning, at the 5'-terminus and at the 3'-terminus, respectively.

U6 upstream consists of the RNA sequence as set forth in SEQ ID NO: 8, and the nucleic acid encoding the U6 upstream consists of the DNA sequence as set forth in SEQ ID NO: 17. U6 downstream consists of the RNA sequence as set forth in SEQ ID NO: 9, and the nucleic acid encoding the U6 downstream consists of the DNA sequence as set forth in SEQ ID NO: 18.

The obtained plasmids are collectively referred to as "pSUPERneo_U6ADg." The constructed pSUPERneo_U6ADg plasmid was amplified using the *Escherichia coli* strain DH5$\alpha$ or the *Escherichia coli* strain Stbl3 by the same method as that of Example 1.

An outline of the DNA sequences encoding individual guide RNAs is shown in Table 1.

Besides, guide RNA expression plasmids used as comparative examples were produced in the same manner as that described above, using DNA sequences (as shown below) encoding the guideRNAs.

< Names of guide RNA sequences and sequence numbers of DNAs encoding those guide RNA sequences (comparative examples) >

U6 upstream-ASR(24)-ARR(40)add-U6 downstream: SEQ ID NO: 34
U6 upstream-ASR(24)-ARR(40)-U6 downstream: SEQ ID NO: 38

The DNA sequence comprises a purine base (G or A) preferable for the transcription start point of polIII on the 5'-terminal side, and a terminator sequence of polIII on the 3'-terminal side. Moreover, the DNA sequence comprises the restriction enzyme BglII recognition sequence (AGATCT) and the restriction enzyme HindII recognition sequence (AAGCTT), which are used in vector cloning, at the 5'-terminus and at the 3'-terminus, respectively.

An outline of the DNA sequences encoding individual guide RNAs is shown in Table 1.

[Table 1]

| | Names of guide RNAs | 5'- | Linker | ASR | ARR | Linker | Linker | 3'- | SEQ ID NO: |
|---|---|---|---|---|---|---|---|---|---|
| Comp. Ex. | ASR(24)-ARR(16)add | - | - | 24nt Rluc | 16nt | add 20nt | - | - | 28 |
| Ex. 1 | ASR(24)-ARR(16)add-3Gq | - | - | | | | - | 3Gq | 19 |
| Ex. 1 | ASR(24)-ARR(16)add-BoxB | - | - | | | | UCU | BoxB | 20 |
| Ex. 1 | BoxB-ASR(24)-ARR(16)add-3Gq | BoxB | UCU | | | | - | 3Gq | 21 |
| Ex. 2 | U6 upstream-ASR(24)-ARR (16)add-U6 downstream | U6 up | - | | | | - | U6 down | 25 |
| Comp. Ex. | ASR(24)-ARR(16) | - | - | | 16nt | - | - | - | 29 |
| Ex. 1 | ASR(24)-ARR(16)-3Gq | - | - | | | | - | 3Gq | 22 |
| Ex. 1 | ASR(24)-ARR(16)-BoxB | - | - | | | | UCU | BoxB | 23 |
| Ex. 1 | BoxB-ASR(24)-ARR(16)-3Gq | BoxB | UCU | | | | - | 3Gq | 24 |
| Ex. 2 | U6 upstream-ASR(24)-ARR (16)-U6 downstream | U6 up | - | | | | - | U6 down | 26 |
| Comp. Ex. | ASR(24)-ARR(40)add | - | - | | 40nt | add 20nt | - | - | 30 |
| Comp. Ex. | ASR(24)-ARR(40)add-3Gq | - | - | | | | - | 3Gq | 31 |
| Comp. Ex. | ASR(24)-ARR(40)add-BoxB | - | - | | | | UCU | BoxB | 32 |
| Comp. Ex. | BoxB-ASR(24)-ARR(40)add-3Gq | BoxB | UCU | | | | - | 3Gq | 33 |
| Comp. Ex. | U6 upstream-ASR(24)-ARR (40)add-U6 downstream | U6 up | - | | | | - | U6 down | 34 |
| Control | ASR(24)-ARR(40) | - | - | | 40nt | - | - | - | 27 |
| Comp. Ex. | ASR(24)-ARR(40)-3Gq | - | - | | | | - | 3Gq | 35 |
| Comp. Ex. | ASR(24)-ARR(40)-BoxB | - | - | | | | UCU | BoxB | 36 |
| Comp. Ex. | BoxB-ASR(24)-ARR(40)-3Gq | BoxB | UCU | | | | - | 3Gq | 37 |
| Comp. Ex. | U6 upstream-ASR(24)-ARR (40)-U6 downstream | U6 up | - | | | | - | U6 down | 38 |

Example 3: Production of reporter mRNA expression plasmid for detecting intracellular target RNA-editing activity

Renilla luciferase (Rluc) mRNA was used as a reporter mRNA for detecting an intracellular target RNA-editing activity. The reporter Rluc mRNA (Rluc-W104X) for detecting an intracellular target RNA-editing activity was designed based on a Rluc mRNA sequence encoded by a Rluc gene incorporated in a psiCHECK-2 vector (Promega, Catalog No. C8021). Specifically, Rluc-W104X was designed such that the 311th G of UGG encoding Trp that is the 104th amino acid of Rluc was substituted with A, and that the translation termination codon (UAG) was encoded, instead of Trp. A Rluc-W104X expression plasmid was produced by substituting the DNA sequence (SEQ ID NO: 40) comprising the mutation point G311A with a psiCHECK-2 vector (Promega, Catalog No. C8021), using the restriction enzymes DraIII (on the 5'-terminal side) and AatII (on the 3'-terminal side). The thus obtained plasmid is referred to as "psiCHECK-2_Rluc-W104X." The constructed psiCHECK-2_Rluc-W104X vector was amplified using the *Escherichia coli* strain DH5α or the *Escherichia coli* strain Stbl3 by the same method as that of Example 1.

Example 4: Production of ADAR2 expression plasmid

An ADAR2 expression plasmid was produced by inserting the DNA sequence (SEQ ID NO: 11) encoding ADAR2 (SEQ ID NO: 12) into the site of the restriction enzymes EcoRI (on the 5'-terminal side) and BamHI (on the 3'-terminal side) located downstream of the CMV-derived promoter of a pAAV-CMV vector contained in AAVpro(registered trademark) Helper Free System (Takara Bio, Inc., Catalog No. 6230), using the restriction enzymes EcoRI (on the 5'-terminal side) and BglII (on the 3'-terminal side). The obtained plasmid is referred to as "pAAV-CMV-ADAR2." In the inserted DNA sequence, a Kozak sequence was disposed upstream the translation initiation codon, and a termination codon was disposed downstream of the ADAR2 gene. The constructed pAAV-CMV-ADAR2 plasmid was amplified using the *Escherichia coli* strain DH5α or the *Escherichia coli* strain Stbl3.

Example 5: Transfection

Transfection 1 (Transfection performed for luciferase assay whose results are shown in Figures 2 and 3, and for studies regarding RNA-editing efficiency according to Sanger sequencing whose results are shown in Table 2)

The human embryonic kidney-derived cell line HEK293 (ATCC Catalog No. CRL-1573) was cultured in a Dulbecco's Modified Eagle Medium (DMEM; Thermo Fisher Scientific, Catalog No. 10569-010) supplemented with 5% fetal bovine serum (FBS). The cultured cells were seeded on a 96-well plate at a cell density of $2.5 \times 10^4$ cells/100 μL, and were then cultured in the presence of 5% $CO_2$ at 37°C overnight. On the following day, the reporter expression plasmid (psiCHECK-2_Rluc-W104X) for detecting an intracellular target RNA-editing activity produced in Example 3, the ADAR2 expression plasmid (pAAV-CMV-ADAR2) produced in Example 4, and the guide RNA expression plasmids produced in Examples 1 and 2 (the after-mentioned three types of pSUPERneo_H1ADg and 1 type of pSUPERneo_U6ADg), and a carrier plasmid were mixed with one another at a weight ratio of 1 : 35 : 35 : 9, respectively, to result in a total amount of 100 ng/well. As such a carrier plasmid, pHelper Vector (Takara Bio, Inc., Catalog No. 6230) was used. Using Lipofectamine 3000 Transfection Reagent (Thermo Fisher Scientific, Catalog No. L3000015), the HEK293 cells were transfected with the above-mixed plasmid.

< Names of guide RNAs expressed by pSUPERneo_H1ADg or pSUPERneo_U6ADg >

ASR(24)-ARR(16)add-3Gq
ASR(24)-ARR(16)add-BoxB
U6 upstream-ASR(24)-ARR(16)add-U6 downstream
BoxB-ASR(24)-ARR(16)add-3Gq

For each plasmid, an experiment was carried out with every 3 wells. After completion of the transfection, the resulting cells were cultured in the presence of 5% $CO_2$ at 37°C, and on Days 3 and 6 of the culture, the cells were harvested, and were then subjected to the detection of an RNA-editing activity according to luciferase assay described in Example 6 and the studies of RNA-editing efficiency according to Sanger sequencing described in Example 7.

Guide RNA expression plasmids used as controls or comparative examples were also prepared in the same manner as that of Transfection 1, and were then transfected into HEK293 cells.

Transfection 2 (Transfection performed for luciferase assay whose results are shown in Figure 4, and for studies regarding RNA-editing efficiency according to Sanger sequencing whose results are shown in Table 2)

The reporter expression plasmid (psiCHECK-2_Rluc-W104X) for detecting an intracellular target RNA-editing activity, the ADAR2 expression plasmid (pAAV-CMV-ADAR2), and the 8 types of guide RNA expression plasmids produced in Examples 1 and 2 (the after-mentioned 6 types of pSUPERneo_H1ADg and 2 types of pSUPERneo_U6ADg), and a carrier plasmid (pHelper Vector) were mixed with one another at a weight ratio of 1 : 30 : 40 : 9, respectively, to result in a total amount of 100 ng/well. Transfection of the mixed plasmid into the HEK293 cells were was carried out by the same method as that of Transfection 1. Besides, the cells were seeded on the plate at a cell density of $2.0 \times 10^4$ cells/100 $\mu$L. After completion of the transfection, the cells were harvested on Day 3 of the culture.

< Names of guide RNAs expressed by pSUPERneo_H1ADg or pSUPERneo_U6ADg >

ASR(24)-ARR(16)add-3Gq
ASR(24)-ARR(16)add-BoxB
U6 upstream-ASR(24)-ARR(16)add-U6 downstream
BoxB-ASR(24)-ARR(16)add-3Gq
ASR(24)-ARR(16)-3Gq
ASR(24)-ARR(16)-BoxB
U6 upstream-ASR(24)-ARR(16)-U6 downstream
BoxB-ASR(24)-ARR(16)-3Gq

Guide RNA expression plasmids used as controls or comparative examples were also used in transfection in the same manner as that of Transfection 2.

Example 6: Detection of RNA-editing activity according to luciferase assay

The firefly luciferase luminescence intensity (Fluc) and the Renilla luciferase luminescence intensity (Rluc) of the cells transfected in Example 5 in each well were measured using Dual-Glo(registered trademark) Luciferase Assay System (Promega, Catalog No. E2940) and EnVision (PerkinElmer) according to protocols included therewith.

In Figure 2 and Figure 3, the Rluc/Fluc value was calculated to correct transfection efficiency among the samples, and the Rluc/Fluc value of each guide RNA sample was shown as a relative value to the Rluc/Fluc value of a guide RNA used as a control that was set to be 1. In the bar graph, the arithmetic mean of the 3 wells was indicated with an error bar and a standard deviation. The same tendency was shown from two times of trials, and the results of representative examples are shown in Figure 2 (Day 3 of the culture) and

Figure 3 (Day 6 of the culture).

The correspondence relationships of individual numbers #1 to #11 and the names of guide RNAs, which are shown in Figure 2 and Figure 3, are shown below. The following names of guide RNAs correspond to the "Names of guide RNAs" shown in Table 1.

#1 ASR(24)-ARR(40)
#2 ASR(24)-ARR(40)-3Gq
#3 ASR(24)-ARR(40)add-3Gq
#4 ASR(24)-ARR(16)add-3Gq
#5 ASR(24)-ARR(40)-BoxB
#6 ASR(24)-ARR(40)add-BoxB
#7 ASR(24)-ARR(16)add-BoxB
#8 U6 upstream-ASR(24)-ARR(40)-U6 downstream
#9 U6 upstream-ASR(24)-ARR(40)add-U6 downstream
#10 U6 upstream-ASR(24)-ARR(16)add-U6 downstream
#11 BoxB-ASR(24)-ARR(16)add-3Gq

In Figure 4, the arithmetic means of the Rluc/Fluc values obtained in independent 3 trials are shown using a bar graph. In addition, the plot indicates the arithmetic means of the Rluc/Fluc values of the samples in each trial, and the error bar indicates a standard deviation.

The correspondence relationships of individual numbers #1 to #20 and the names of guide RNAs, which are shown in Figure 4, are shown below. The following names of guide RNAs correspond to the "Names of guide RNAs" shown in Table 1.

#1 ASR(24)-ARR(16)add
#2 ASR(24)-ARR(16)add-3Gq
#3 ASR(24)-ARR(16)add-BoxB
#4 BoxB-ASR(24)-ARR(16)add-3Gq
#5 U6 upstream-ASR(24)-ARR(16)add-U6 downstream
#6 ASR(24)-ARR(16)
#7 ASR(24)-ARR(16)-3Gq
#8 ASR(24)-ARR(16)-BoxB
#9 BoxB-ASR(24)-ARR(16)-3Gq
#10 U6 upstream-ASR(24)-ARR(16)-U6 downstream
#11 ASR(24)-ARR(40)add
#12 ASR(24)-ARR(40)add-3Gq
#13 ASR(24)-ARR(40)add-BoxB
#14 BoxB-ASR(24)-ARR(40)add-3Gq
#15 U6 upstream-ASR(24)-ARR(40)add-U6 downstream
#16 ASR(24)-ARR(40)
#17 ASR(24)-ARR(40)-3Gq
#18 ASR(24)-ARR(40)-BoxB
#19 BoxB-ASR(24)-ARR(40)-3Gq
#20 U6 upstream-ASR(24)-ARR(40)-U6 downstream

In the reporter (Rluc-W104X) for detecting an intracellular target RNA-editing activity, UGG encoding the 104th Trp of Rluc is substituted with the termination codon (UAG). Accordingly, if the editing of the A of the target RNA to I is induced in a cell by the ADAR protein and the guide RNA, the mutated translation termination codon is converted to Trp when it is translated from the mRNA, and the luminescence of Renilla luciferase is recovered. Specifically, it is shown that the higher the luminescence intensity, the higher the RNA-editing activity that can be obtained.

As shown from Figure 2 to Figure 4, a guide RNA comprising a functional nucleotide sequence and a short-chain ADAR-recruiting nucleotide sequence ARR(16) exhibited a higher RNA-editing activity than those of controls (i.e. #1 in Figures 2 and 3, and #16 in Figure 4) (a guide RNA comprising the ADAR-recruiting nucleotide sequence ARR(40)), regardless of the presence or absence of a linker nucleotide sequence. On the other hand, when a functional nucleotide sequence was added to such a control via a linker nucleotide sequence, the RNA-editing activity was decreased, compared with the case of not using a linker nucleotide sequence.

Example 7: Studies regarding RNA-editing efficiency according to Sanger sequencing

The transfected cells obtained in Transfections 1 and 2 of Example 5 were each harvested, and total RNA was then extracted and purified from the harvested cells using QIAshredder (QIAGEN, Catalog No. 79656), RNeasy Mini Kit (QIAGEN, Catalog No. 74106), and RNase-free DNase Set (QIAGEN, Catalog No. 79254), according to protocols included therewith. The purified RNA was subjected to a reverse transcription reaction, using SuperScript(registered trademark) VILO cDNA Synthesis kit (Thermo Fisher Scientific, Catalog No. 11754-250), according to protocols included therewith, so as to obtain a cDNA. Using the obtained cDNA as a template and PrimeSTAR(registered trademark) GXL DNA Polymerase (Takara Bio, Inc., Catalog No. R050A), a PCR reaction was carried out employing the following primers for amplifying a fragment containing an edition point, according to protocols included therewith.

< Primers used in the experiment shown in Table 2 >

Forward primer: GGTAACGCTGCCTCCAGC (SEQ ID NO: 41)
Reverse primer: TGTAGTTGCGGACAATCTGG (SEQ ID NO: 42)

< Primers used in the experiment shown in Table 3 >

Forward primer: CTCGCTGCAAGCAAATGAAC (SEQ ID NO: 43)
Reverse primer: TCGTCCCAGGACTCGATCAC (SEQ ID NO: 44)

The PCR amplified fragment was purified using ExoSAP-IT Express PCR Cleanup Reagents (Thermo Fisher Scientific, Catalog No. 75001.200.UL) according to protocols included therewith, and the resulting product, together with the forward primer used in the PCR amplification, was subjected to a Sanger sequencing reaction using 3730×1 (XL) DNA Analyzer (Applied Biosystems).

In Table 2, the DNA chromatogram (filename extension: .ab1) obtained in the Sanger sequencing reaction was analyzed using QSVanalyzer software (Bioinformatics, 2009, Vol. 25, pp. 3244-3250), and the abundance ratio of G (G/(G+A) × 100), which was obtained when the signal strength of the guanine (G) of unedited Rluc-W104X was set at 0, the signal strength of the adenine (A) thereof was set at 1, the signal strength of G of Rluc was set at 1, and the signal strength of A thereof is set at 0, was defined as RNA-editing efficiency (%). The same tendency was shown from two times of trials, and the results of representative examples are shown in Figure 2.

[Table 2]

| Functional nucleotide sequences | Names of guide RNAs | RNA-editing efficiency (%) | |
|---|---|---|---|
| | | Day 3 | Day 6 |
| Control | ASR(24)-ARR(40) | 40.7 | 53.4 |
| Gq | ASR(24)-ARR(40)-3Gq | 43.2 | 50.9 |
| | ASR(24)-ARR(40)add-3Gq | 17.1 | 28.7 |
| | ASR(24)-ARR(16)add-3Gq | 65.3 | 70.6 |
| BoxB | ASR(24)-ARR(40)-BoxB | 40.9 | 50.5 |
| | ASR(24)-ARR(40)add-BoxB | 22.0 | 38.8 |
| | ASR(24)-ARR(16)add-BoxB | 81.6 | 82.0 |
| U6 insert | U6 upstream-ASR(24)-ARR(40)-U6 downstream | 44.5 | 61.5 |
| | U6 upstream-ASR(24)-ARR(40)add-U6 downstream | 21.8 | 44.4 |
| | U6 upstream-ASR(24)-ARR(16)-U6 downstream | 55.7 | 66.1 |
| BoxB/Gq | BoxB-ASR(24)-ARR(16)add-3Gq | 71.0 | 70.1 |

The guide RNA used as a control (a guide RNA comprising an ADAR-recruiting nucleotide sequence ARR(40)) exhibited an RNA-editing efficiency of 40.7% on day 3 after the transfection. On the other hand, the guide RNA of the present invention (a guide RNA comprising a functional nucleotide sequence and a short-chain ADAR-recruiting nucleotide sequence ARR(16)) exhibited a high RNA-editing efficiency of approximately 60% to 80%.

In Table 3, the DNA chromatogram (filename extension: .ab1) obtained in the Sanger sequencing reaction was analyzed using the QSVanalyzer software, and the signal strength ratio of guanine (G) (G/(G+A) × 100) was defined as an RNA-editing efficiency (%). The same tendency was shown from two times of trials, and the results of representative examples are shown below.

[Table 3]

| Names of guide RNAs | Combinations of ARRs and linkers | Functional nucleotide sequences | RNA-editing efficiency (%) |
|---|---|---|---|
| ASR(24)-ARR(16)add | ARR(16)add | - | 65.6 |
| ASR(24)-ARR(16)add-3Gq | | 3Gq | 61.1 |
| ASR(24)-ARR(16)add-BoxB | | BoxB | 73.5 |
| BoxB-ASR(24)-ARR(16)add-3Gq | | BoxB/3Gq | 74.4 |
| U6 upstream-ASR(24)-ARR(16) add-U6 downstream | | U6 insert | 75.8 |

(continued)

| Names of guide RNAs | Combinations of ARRs and linkers | Functional nucleotide sequences | RNA-editing efficiency (%) |
|---|---|---|---|
| ASR(24)-ARR(16) | ARR(16) | - | 68.4 |
| ASR(24)-ARR(16)-3Gq | | 3Gq | 62.8 |
| ASR(24)-ARR(16)-BoxB | | BoxB | 76.7 |
| BoxB-ASR(24)-ARR(16)-3 Gq | | BoxB/3Gq | 70.6 |
| U6upstream-ASR(24)-ARR(16)-U6 downstream | | U6 insert | 76.8 |
| ASR(24)-ARR(40)add | ARR(40)add | - | 26.6 |
| ASR(24)-ARR(40)add-3Gq | | 3Gq | 31.0 |
| ASR(24)-ARR(40)add-BoxB | | BoxB | 32.8 |
| BoxB-ASR(24)-ARR(40)add-3Gq | | BoxB/3Gq | 43.2 |
| U6 upstream-ASR(24)-ARR(40) add-U6 downstream | | U6 insert | 32.9 |
| ASR(24)-ARR(40) | ARR(40) | - | 44.6 |
| ASR(24)-ARR(40)-3Gq | | 3Gq | 43.2 |
| ASR(24)-ARR(40)-BoxB | | BoxB | 48.1 |
| BoxB-ASR(24)-ARR(40)-3Gq | | BoxB/3Gq | 63.7 |
| U6upstream-ASR(24)-ARR(40)-U6 downstream | | U6 insert | 46.3 |

The guide RNA used as a control (ASR(24)-ARR(40)) (a guide RNA comprising an ADAR-recruiting nucleotide sequence ARR(40)) exhibited an RNA-editing efficiency of 44.6%. On the other hand, a guide RNA comprising a functional nucleotide sequence and a short-chain ADAR-recruiting nucleotide sequence ARR(16)) exhibited a high RNA-editing efficiency of approximately 60% to 75%, compared with the guide RNA used as a control.

Example 8: Studies regarding RNA-editing efficiency targeting firefly luciferase (Fluc) mRNA

Whether the guide RNA of the present invention exhibits a high RNA-editing efficiency even to a different target RNA was examined.

A guide RNA expression plasmid having, as an editing target, the 74th nucleotide adenosine in the coding region of firefly luciferase (Fluc) incorporated in a psiCHECK-2 vector (Promega, Catalog No. C8021) was produced in the same manner as those of Examples 1 and 2. ASRf(24) indicates an antisense nucleotide sequence consisting of 24 nucleotides complementary to a Fluc reporter mRNA comprising the adenosine as an editing target, and consists of an RNA nucleotide sequence encoded by a DNA nucleotide sequence from the nucleotide numbers 9 to 32 as set forth in SEQ ID NO: 45. The DNA sequences encoding the guide RNAs used in the production are as follows.

< Names of guide RNAs and sequence numbers of DNAs encoding guide RNAs >

ASRf(24)-ARR(16)add-3Gq: SEQ ID NO: 45
ASRf(24)-ARR(16)add-BoxB: SEQ ID NO: 46
U6 upstream-ASRf(24)-ARR(16)add-U6 downstream: SEQ ID NO: 47

The DNA sequence comprises a purine base (G or A) preferable for the transcription start point of polIII on the 5'-terminal side, and a terminator sequence of polIII on the 3'-terminal side. In addition, the DNA sequence comprises the sequence (AGATC) comprising the restriction enzyme BglII protruding end and the restriction enzyme HindIII recognition sequence (AAGCTT), which are used in vector cloning, at the 5'-terminus and at the 3'-terminus, respectively.

The obtained plasmids are collectively referred to as "pSUPERneo_H1ADg_ASRf24."

Besides, guide RNA expression plasmids used as a control and as comparative examples were also produced in the same manner as that described above, using DNA sequences encoding guide RNAs (as shown below).

< Name of guide RNA and sequence number of DNA encoding guide RNA (control) >

ASRf(24)-ARR(40): SEQ ID NO: 48

< Names of guide RNAs and sequence numbers of DNAs encoding guide RNAs (comparative examples) >

ASRf(24)-ARR(16)add: SEQ ID NO: 49
ASRf(24)-ARR(16): SEQ ID NO: 50

The DNA sequence comprises a purine base (G or A) preferable for the transcription start point of polIII on the 5'-terminal side, and a terminator sequence of polIII on the 3'-terminal side. In addition, the DNA sequence comprises the sequence (AGATC) comprising the restriction enzyme BglII protruding end and the restriction enzyme HindIII recognition sequence (AAGCTT), which are used in vector cloning, at the 5'-terminus and at the 3'-terminus, respectively.

In the same manner as that of Example 5 (Transfection 2), HEK293 cells were transfected with the Fluc reporter expression plasmid (psiCHECK-2 (Promega, C8021)), the ADAR2 expression plasmid (pAAV-CMV-ADAR2) produced in Example 4, and the above-described guide RNA expression plasmid (pSUPERneo_H1ADg_ASRf24). The guide RNA expression plasmids used as controls and comparative examples were also used in the transfection.

A PCR reaction was carried out in the same manner as that of Example 7 (in the case shown in Table 3), and a Sanger sequencing reaction was then carried out using sequencing primers. The RNA-editing efficiency targeting the Fluc reporter mRNA was calculated from the chromatogram of the Sanger sequencing reaction. The primers used in the PCR reaction and the Sanger sequencing reaction of the present test are shown below.

Forward primer Fluc: GGCCGATGCTAAGAACATTAAGAAG (SEQ ID NO: 51)
Reverse primer Fluc: CCACGGTAGGCTGAGAAATG (SEQ ID NO: 52)
Sequencing primer: CTCCGATGAACAGGGCGC (SEQ ID NO: 53)

The obtained results are shown in Table 4.

[Table 4]

| Names of guide RNAs | Combinations of ARRs and linkers | Functional nucleotide sequences | RNA-editing efficiency (%) |
|---|---|---|---|
| ASRf(24)-ARR(16)add | ARR(16)add | - | 8.1 |
| ASRf(24)-ARR(16)add-3Gq | | 3Gq | 15.2 |
| ASRf(24)-ARR(16)add-BoxB | | BoxB | 4.3 |
| U6 upstream-ASRf(24)-ARR(16) add-U6 downstream | | U6 insert | 3.1 |
| ASRf(24)-ARR(16) | ARR(16) | - | 9.0 |
| ASRf(24)-ARR(40) | ARR(40) | - | 1.8 |

The guide RNAused as a control (ASRf(24)-ARR(40)) (a guide RNA comprising an ADAR-recruiting nucleotide sequence ARR(40)) exhibited an RNA-editing efficiency of 1.8% on 3 days after the transfection. On the other hand, the guide RNA comprising a functional nucleotide sequence and a short-chain ADAR-recruiting nucleotide sequence ARR(16) exhibited a high RNA-editing efficiency of approximately 3% to 15%, compared with the guide RNA used as a control.

Reference Example 1: Studies regarding stability of guide RNA *in vitro*

The stability of a guide RNA *in vitro* can be evaluated by making a comparison in terms of the remaining amount of the synthesized guide RNA. As the remaining amount of a guide RNA of interest increases, it means that the stability of the guide RNA is high.

Specifically, a guide RNA is synthesized by *in vitro* transcription from a double-stranded DNA, the obtained guide RNA is then treated with exoribonuclease, and the remaining guide RNA is then quantified.

The double-stranded DNA is not particularly limited, as long as the guide RNA of the present invention can be synthesized by *in vitro* transcription from the double-stranded DNA. The double-stranded DNA may be, for example, a double-stranded DNA comprising a region encoding a guide RNA downstream of a promoter region. It is also possible to add a sequence enhancing transcription efficiency to the double-stranded DNA. For example, it is possible to add GG or GGG to the 5'-terminus of an RNA sequence to be transcribed. The promoter region is not particularly limited, either, and for example, a region comprising a T7 RNA polymerase, T3 RNA polymerase, or SP6 RNA polymerase-binding region can be used.

The method of producing a double-stranded DNA to be used in the *in vitro* transcription is not particularly limited. For example, a double-stranded DNA obtained by synthesizing two oligo DNAs having a complementary relationship to each other and then subjecting the two oligo DNAs to an ordinary annealing reaction, and a double-stranded DNA produced from a plasmid DNA, etc. used as a template according to a PCR method, can be used. Depending on the sequence of a double-stranded DNA, such a double-stranded DNA can be prepared according to a restriction enzyme treatment, a Fill-in reaction involving Klenow Fragment, and the like. In order to obtain a double-stranded DNA having such a purity that can be used in the *in vitro* transcription reaction, the produced double-stranded DNA is separated and/or purified according to, for example, agarose gel electrophoresis, polyacrylamide gel electrophoresis, phenol/chloroform extraction, or ethanol precipitation, and the resultant can be then used in the *in vitro* transcription.

The *in vitro* transcription from the double-stranded DNAis not particularly limited, and it can be carried out using, for example, T7-Scribe Standard RNA IVT KIT (CELLSCRIPT, Catalog No. C-AS2607), MAXIscript T7 Transcription Kit (Thermo Fisher, Catalog No. AM1312), and T7 RNA polymerase (Takara Bio, Inc., Catalog No. 2540A), according to protocols included therewith. Purification of an RNA from the reaction solution obtained after completion of the *in vitro* transcription can be carried out according to an ordinary method. For instance, the reaction solution is treated with DNase, an RNA is then purified by phenol/chloroform extraction and ethanol precipitation, and the purified RNA is further separated according to gel electrophoresis, so that an RNA can be purified from a band at a desired position. As such gel electrophoresis, denaturing polyacrylamide gel electrophoresis can be, for example, used. For purification of the RNA from the gel, filter filtration and gel filtration can be used.

As exoribonuclease used in the exoribonuclease treatment of a guide RNA, for example, RNaseR (Lucigen, Catalog No. RNR07250) or RNaseR (BioVision, Catalog No. M1228-500) can be used. The buffers and conditions used in the exoribonuclease treatment are not particularly limited. For instance, 0.1 to 10 U RNaseR can be used with respect to 250 ng of a guide RNA, and the reaction can be carried out in 1× RNase R Reaction Buffer (20 mM Tris-HCl (pH8.0), 100 mM KCl, and 0.1 mM $MgCl_2$) under conditions of 37°C and 10 minutes to 2 hours. As described later, in the case of using a radioisotope (RI) labeled RNA or a fluorescently labeled RNA, the RNA can also be treated using a cell extraction solution, instead of exoribonuclease.

Quantification of the RNA remaining after the exoribonuclease treatment can be carried out according to an ordinary method. For example, the solution obtained after completion of the exoribonuclease treatment reaction is separated by gel electrophoresis, and the gel is then stained, so that a remaining RNA can be quantified. Herein, for the gel electrophoresis, denaturing polyacrylamide gel electrophoresis can be used. For the staining of the gel, ethidium bromide or SYBR Gold Nucleic Acid Gel Stain (Thermo Fisher, Catalog No. S11494) can be used. As another method of quantifying a remaining RNA, a bioanalyzer (manufactured by Agilent; 2100 BioAnalyzer) can also be used.

As another method of quantifying an RNA remaining after the exoribonuclease treatment, a method of quantifying an RNA labeled with RI, fluorescence, etc. can also be applied. The RI used in the labeling is not particularly limited, as long as it is able to label the RNA. For example, $\gamma$-$^{32}$P-ATP can be used. As a labeling method with RI, for example, the 5'-terminus of an RNA is dephosphorylated using a dephosphorylation enzyme, and the RNA is then purified by phenol/chloroform extraction, and thereafter, a phosphorylation reaction can be carried out using polynucleotide kinase in the presence of [$\gamma$-$^{32}$P]-ATP. As such a dephosphorylation enzyme, for example, Antarctic Phosphatase (New England Biolabs, Catalog No. M0289S) can be used. As ATP, for example, [$\gamma$-$^{32}$P]-ATP (PerkinElmer, Catalog No. BLU002Z250UC) can be used. As polynucleotide kinase, for example, T4 polynucleotide kinase (New England Biolabs, Catalog No. M0201S) can be used. The fluorescent dye used in the labeling is not particularly limited, as long as it is able to label the RNA. The fluorescently labeling method may be, for example, a method of labeling the 5'-terminus, and 5' EndTag™ Nucleic Acid Labeling System (Vector Laboratories, Catalog No. MB-9001) can be used. The thus RI-labeled or fluorescently labeled RNA is subjected to an exoribonuclease treatment according to the same method as that described above, and after completion of the reaction, the solution is separated by gel electrophoresis. After completion of the separation, radioactivity or fluorescence intensity on the gel is detected using an image analyzer, so that the remaining RNA can be quantified. As such an image analyzer, for example, Fluoro Image Analyzer (Fujifilm, Catalog No. FLA-7000) can be used.

An example of a detailed method of studying the stability of a guide RNA *in vitro* by making a comparison in terms of the remaining amount of the guide RNA will be described below, but the example is not limited thereto.

Using, as a template, a plasmid DNA (wherein, for example, the guide RNA expression plasmid (pSUPERneo_H1_gRNA or pSUPERneo_U6_gRNA) described in Example 1 or 2 can be used), a PCR reaction is carried out using a T7 RNA polymerase-binding sequence, a sequence enhancing transcription activity, a forward primer comprising a sequence encoding a part of a guide RNA, a reverse primer comprising a sequence encoding a part of the guide RNA, and Prime Star GXL (Takara Bio, Inc., Catalog No. R050B). Thereafter, the amplified PCR product is purified by phenol/chloroform extraction and ethanol precipitation to obtain a template DNA. Using the obtained DNA as a template, *in vitro* transcription is carried out (wherein the reaction conditions are, for example, 37°C and 3 hours) employing T7-Scribe Standard RNA IVT KIT (manufactured by CELLSCRIPT) according to protocols included therewith, so as to synthesize an RNA. Thereafter, DNase is added to perform a reaction of decomposing the template DNA, and an RNA is then purified by phenol/chloroform extraction and ethanol precipitation. After that, the obtained RNA is purified by 8 M Urea PAGE (8%), and is then extracted by crushing and/or immersion. The extract is purified using a 0.22-μm filter and gel filtration, thereby preparing various types of guide RNAs.

The guide RNA is subjected to a dephosphorylation reaction (e.g. 37°C, 1 hour), using Antarctic Phosphatase (New England Biolabs, Catalog No. M0289S). After completion of the reaction, the RNA is purified by phenol-chloroform extraction and ethanol precipitation.

[$\gamma$-$^{32}$P]-ATP (PerkinElmer#BLU002Z250UC) is added to the RNA after completion of the dephosphorylation, and a phosphorylation reaction (e.g. 37°C, 30 minutes) is then carried out using 1 U/μl (final concentration) T4 polynucleotide kinase (New England Biolabs, Catalog No. M0201S). After completion of the reaction, the RNA is purified by ethanol precipitation. The RNA is dissolved in 80% formamide, and is then cut out by 8 M Urea 8% polyacrylamide gel electrophoresis (PAGE), and the RNA is then purified by the same method as that described above. The radioactivity of the obtained RNA is measured using a liquid scintillation counter (PerkinElmer, Catalog No. Tri-Carb 2910 TR).

For example, 0.1 to 10 U RNase R is added to the radiolabeled guide RNA, and a decomposition reaction is then carried out (e.g. 37°C, 10 minutes to 2 hours). After completion of the reaction, the solution is separated by 8 M Urea 8% polyacrylamide gel electrophoresis (PAGE). The surface of the gel is covered with a wrap, and a Fuji imaging plate (Fujifilm, Catalog No.) is then placed thereon, followed by performing light exposure for 6 hours or more. After completion of the light exposure, the imaging plate is analyzed using an image analyzer (Fujifilm, Catalog No. FLA-7000). The remaining RNA is quantified from the obtained band.

Reference Example 2: Studies regarding stability of guide RNA in cells

The stability of a guide RNA in cells can be evaluated by making a comparison in terms of the amount of a guide RNA remaining after inhibition of the transcription. As the amount of a remaining guide RNA of interest increases, it means that the guide RNA has high stability.

Specifically, after a guide RNA expression plasmid has been transfected into the cultured cells and then the guide RNA has been sufficiently expressed therein, the transcription of the guide RNA is inhibited by a transcription inhibitor for a certain period of time. Thereafter, the RNA is extracted and purified from the cells, and the remaining guide RNA is then quantified. In another aspect, a guide RNA expression plasmid is transfected into the cultured cells, and a newly transcribed RNA is pulse-labeled. Thereafter, the labeled RNA is recovered, and the remaining guide RNA is then quantified.

The guide RNA expression plasmid is not particularly limited, as long as it is able to express the guide RNA of the present invention. For example, the guide RNA expression plasmid (pSUPERneo_H1ADg or pSUPERneo_U6ADg) described in Examples 1 and 2 can be used. The reporter expression plasmid for detecting an intracellular target RNA-editing activity (psiCHECK-2_Rluc-W104X) described in Example 3 and the ADAR2 expression plasmid (pAAV-CMV-ADAR2) described in Example 4 may be used in the transfection simultaneously with the guide RNA expression plasmid.

The cultured cells are not particularly limited, as long as they are able to express the guide RNA. For example, HEK293 cells can be used.

The guide RNA expression plasmid can be transfected into the cultured cells according to an ordinary method. The transfection method is not particularly limited, and the transfection can be carried out, for example, using Lipofectamine 3000 Transfection Reagent, according to protocols included therewith. The concentration of the guide RNA expression plasmid used in the transfection can be set to be an appropriate concentration, depending on the cultured cells.

The transcription inhibitor is not particularly limited. For example, actinomycin D (Wako Pure Chemical Industries, Ltd., Catalog No. 010-21263), α-amanitin (Wako Pure Chemical Industries, Ltd., Catalog No. 010-22961), etc. can be used. It has been known that actinomycin D binds to a double-stranded DNA and inhibits transcription by an RNA polymerase. It is also possible to use a Tet-inducible promoter, etc., instead of such a transcription inhibitor, so as to transiently express a guide RNA from a guide RNA expression plasmid.

It requires, for example, 24 hours after the transfection, in order to reach a state in which a guide RNA has been sufficiently expressed after the transfection of a guide RNA expression plasmid into the cultured cells, but the required time is not limited thereto.

After addition of the transcription inhibitor, the transfected cells are harvested at any given timing, and the RNA is then purified. Such any given timing for harvesting the cells can be determined, depending on a period of time, in which a highly stable RNA used as a positive control remains. Such any given timing for recovering the cells is not particularly limited, and it may be 30 minutes to 16 hours after addition of the transcription inhibitor.

Extraction and purification of a total RNA comprising the guide RNA from the harvested cells can be carried out according to an ordinary method. The method of extracting the RNA is not particularly limited. For example, a microRNA extraction/purification kit, such as miRNeasy Mini Kit (QIAGEN, Catalog No. 217004), or mirVana miRNA Isolation Kit (Thermo Fisher Scientific, Catalog No. AM1560), or NucleoSpin miRNA (Takara Bio, Inc., Catalog No. U0971B), can be used. When DNA is removed from the extracted total RNA, a kit such as RNase-free DNase Set can be used.

Quantification of the RNA can be carried out according to an ordinary method. For example, using cDNA produced from the extracted RNA as a template, a PCR reaction is carried out according to an ordinary method, and a guide RNA of interest can be quantified. The method of producing cDNA is not particularly limited. For example, poly A is added to a small RNA using Mir-X miRNA First-Strand Synthesis Kit (Takara Bio, Inc., Catalog No. Z8315N) according to protocols included therewith, and thereafter, a reverse transcription reaction is carried out to obtain cDNA. The method of quantifying a guide RNA according to a real-time PCR reaction is not particularly limited. Using the obtained cDNA as a template, a real-time PCR reaction is carried out employing QuantStudio 12K Flex Real-Time PCR System (Thermo Fisher Scientific), and using Mir-X™ miRNA qRT-PCR TB Green(registered trademark) Kit (Takara Bio, Inc., Catalog No. Z8316N) according to protocols included therewith, so that the guide RNA is quantified. The forward primer used in the real-time PCR reaction can be designed based on the sequence of the guide RNA to be quantified, according to a method known to those skilled in the art.

In another aspect, a newly transcribed RNAis pulse-labeled, and the labeled RNA is then recovered, and thereafter, the remaining guide RNA can be quantified. The pulse-labeling reagent is not particularly limited, and for example, 5-bromouridine (BrU) can be used. Specifically, an RNA that is newly generated in cells can be labeled with BrU, using BRIC Kit (MBL Life Sciences, Catalog No. RN1007) according to protocols included therewith. The labeled RNA is recovered by an immunoprecipitation method, and the amount of the guide RNAis quantified according to the above-described PCR method.

An example of a detailed method of studying the stability of a guide RNA in cells by making a comparison in terms of the remaining amount of the guide RNA after inhibition of the transcription will be described below, but the example is not limited thereto.

The reporter expression plasmid for detecting an intracellular target RNA-editing activity (psiCHECK-2_Rluc-W104X), the ADAR2 expression plasmid (pAAV-CMV-ADAR2), and the guide RNA expression plasmid (pSUPERneo_H1ADg or pSUPERneo_U6ADg) (all of which are described in Examples 1 to 4) are transfected into HEK293 cells, using Lipofectamine 3000 Transfection Reagent according to protocols included therewith. Twenty four hours later, a treatment with actinomycin D is carried out. It has been known that actinomycin D binds to a double-stranded DNA and inhibits transcription by an RNA polymerase. The transfected cells are harvested until 30 minutes to 16 hours after inhibition of the transcription, and then, a total RNA comprising the guide RNA is extracted and purified from the harvested cells, using miRNeasy Mini Kit and RNase-free DNase Set, according to protocols included therewith. With regard to the obtained RNA, poly A is added to a small RNA, using Mir-X miRNA First-Strand Synthesis Kit (Takara Bio, Inc., Catalog No. Z8315N), according to protocols included therewith, and thereafter, a reverse transcription reaction is carried out to obtain cDNA. Using the obtained cDNA as a template, a real-time PCR reaction is carried out employing QuantStudio™ 12K Flex Real-Time PCR System (Thermo Fisher Scientific), and using Mir-X miRNA qRT-PCR TB Green (registered trademark) Kit according to protocols included therewith, so that the guide RNA is quantified. The forward primer used in the real-time PCR reaction is designed to have a sequence homologous to the antisense nucleotide sequence of the nucleic acid encoding the guide RNA.

Industrial Applicability

The guide RNA of the present invention is useful for editing a target RNA. A pharmaceutical composition comprising the guide RNA, a nucleic acid encoding the guide RNA, the expression vector of the present invention, or the polypeptide of the present invention, is useful in that it can be used for various diseases including hereditary diseases as typical examples, which can be prevented or treated by editing a target RNA.

Reference Signs List

1: Guide RNA, 10: Antisense nucleotide sequence, 20: Short-chain ADAR-recruiting nucleotide sequence, 30: Functional nucleotide sequence, 40: Target RNA sequence, 50: ADAR, 60: Linker

Sequence Listing Free Text

SEQ ID NOs: 1 to 10: Synthetic RNAs
SEQ ID NOs: 13 to 53: Synthetic DNAs

**Claims**

1. A guide RNA for editing a target RNA sequence, comprising:
an antisense nucleotide sequence complementary to a portion of the target RNA sequence, a short-chain ADAR-recruiting nucleotide sequence, and at least one functional nucleotide sequence.

2. The guide RNA according to claim 1, wherein the antisense nucleotide sequence, the short-chain ADAR-recruiting nucleotide sequence, and the at least one functional nucleotide sequence are connected with one another, in this order, from the 5'-terminal side to the 3'-terminal side.

3. The guide RNA according to claim 2, wherein at least one functional nucleotide sequence is further connected with the 5'-terminal side of the antisense nucleotide sequence.

4. The guide RNA according to claim 1, further comprising a linker nucleotide sequence.

5. The guide RNA according to claim 4, wherein at least one functional nucleotide sequence is connected with the short-chain ADAR-recruiting nucleotide sequence via the linker nucleotide sequence.

6. The guide RNA according to claim 5, wherein the antisense nucleotide sequence, the short-chain ADAR-recruiting nucleotide sequence, the linker nucleotide sequence, and the at least one functional nucleotide sequence are connected with one another, in this order, from the 5'-terminal side to the 3'-terminal side.

7. The guide RNA according to claim 6, wherein at least one functional nucleotide sequence is further connected with the 5'-terminal side of the antisense nucleotide sequence.

8. The guide RNA according to any one of claims 1 to 7, wherein the functional nucleotide sequence is a nucleotide sequence associated with the intracellular stabilization and/or intracellular localization of the guide RNA.

9. The guide RNA according to any one of claims 1 to 8, wherein the functional nucleotide sequence(s) are one or more nucleotide sequences selected from a nucleotide sequence that forms a G-quadruplex structure and a nucleotide sequence that forms a stem-loop structure.

10. The guide RNA according to any one of claims 1 to 8, wherein the functional nucleotide sequence is a U6 snRNA sequence.

11. The guide RNA according to claim 9, wherein the functional nucleotide sequence is a nucleotide sequence that forms a 1 to 4 layered G-quadruplex structure.

12. The guide RNA according to claim 9, wherein the functional nucleotide sequence is a nucleotide sequence that forms a stem-loop structure.

13. The guide RNA according to claim 12, wherein the nucleotide sequence that forms a stem-loop structure is a nucleotide sequence derived from a BoxB sequence.

14. The guide RNA according to any one of claims 1 to 13, wherein the functional nucleotide sequence consists of the nucleotide sequence as set forth in SEQ ID NO: 6, 7, 8, or 9, or a nucleotide sequence comprising a deletion, substitution, insertion and/or addition of 1 to 3 nucleotides with respect to the nucleotide sequence as set forth in SEQ ID NO: 6, 7, 8, or 9, and having functions.

15. The guide RNA according to any one of claims 1 to 14, wherein the short-chain ADAR-recruiting nucleotide sequence is a nucleotide sequence consisting of 14 to 34 nucleotides.

16. The guide RNA according to claim 15, wherein the short-chain ADAR-recruiting nucleotide sequence is a sequence consisting of 16 nucleotides.

17. The guide RNA according to claim 15 or 16, wherein the loop portion in the stem-loop structure of the short-chain ADAR-recruiting nucleotide sequence is a nucleotide sequence consisting of 4 or 5 nucleotides.

18. The guide RNA according to any one of claims 15 to 17, wherein the stem portion in the stem-loop structure of the short-chain ADAR-recruiting nucleotide sequence has nucleotides that form a mismatched base pair.

19. The guide RNA according to any one of claims 1 to 18, wherein the short-chain ADAR-recruiting nucleotide sequence consists of the nucleotide sequence as set forth in SEQ ID NO: 1, 2, 3, or 4, or a nucleotide sequence comprising a deletion, substitution, insertion and/or addition of 1 to 3 nucleotides with respect to the nucleotide sequence as set forth in SEQ ID NO: 1, 2, 3, or 4, and forming a stem-loop structure.

20. The guide RNA according to any one of claims 4 to 19, wherein the linker nucleotide sequence is a nucleotide sequence consisting of 15 to 25 nucleotides.

21. A target RNA sequence-editing system, comprising the guide RNA according to any one of claims 1 to 20 and ADAR.

22. A nucleic acid encoding the guide RNA according to any one of claims 1 to 20.

23. An expression vector, comprising a nucleic acid encoding the guide RNA according to any one of claims 1 to 20.

24. The expression vector according to claim 23, further comprising a nucleic acid encoding ADAR.

25. A host cell, into which the expression vector according to claim 23 or 24 is introduced.

26. A method for producing an expression vector, comprising a step of culturing the host cell according to claim 25.

27. A pharmaceutical composition, comprising the expression vector according to claim 23 or 24 and a pharmaceutically acceptable excipient.

28. A pharmaceutical composition, comprising the expression vector according to claim 23, an expression vector containing a nucleic acid encoding ADAR, and a pharmaceutically acceptable excipient.

29. The pharmaceutical composition according to claim 27 or 28, wherein the ADAR is human ADAR1 or human ADAR2

30. The pharmaceutical composition according to claim 28 or 29, wherein the nucleic acid encoding ADAR is a nucleic acid consisting of a nucleotide sequence encoding the amino acid sequence as set forth in SEQ ID NO: 12, or a nucleotide sequence encoding a polypeptide that consists of an amino acid sequence having an identity of 90% or more to the amino acid sequence as set forth in SEQ ID NO: 12 and has an adenosine deaminase activity.

**31.** A method for editing a target RNA, comprising: (i) a step of introducing the guide RNA according to any one of claims 1 to 20 or the nucleic acid according to claim 22 into a cell; (ii) a step of recruiting ADAR by the guide RNA; (iii) a step of forming a complex of a target RNA sequence and the guide RNA; (iv) a step of converting the adenosine of the target RNA sequence to inosine by the ADAR recruited by the guide RNA.

[Figure 1]

(A)

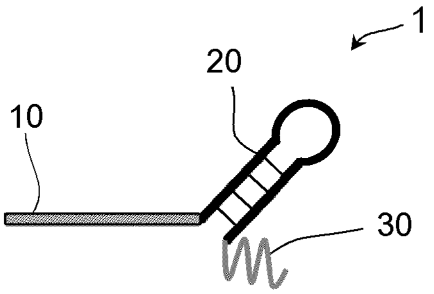


(B)

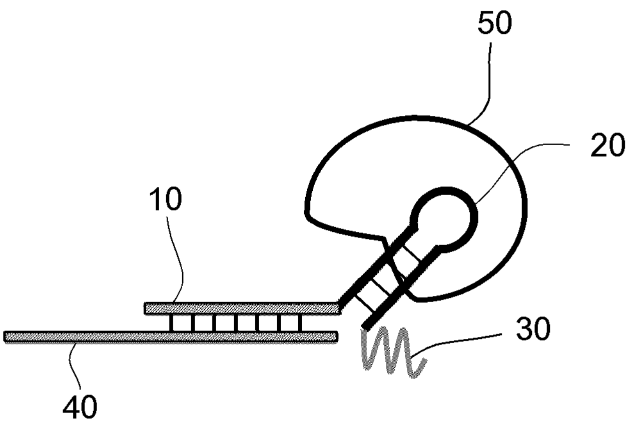


(C)

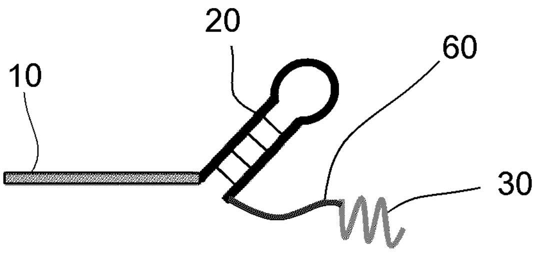

[Figure 2]

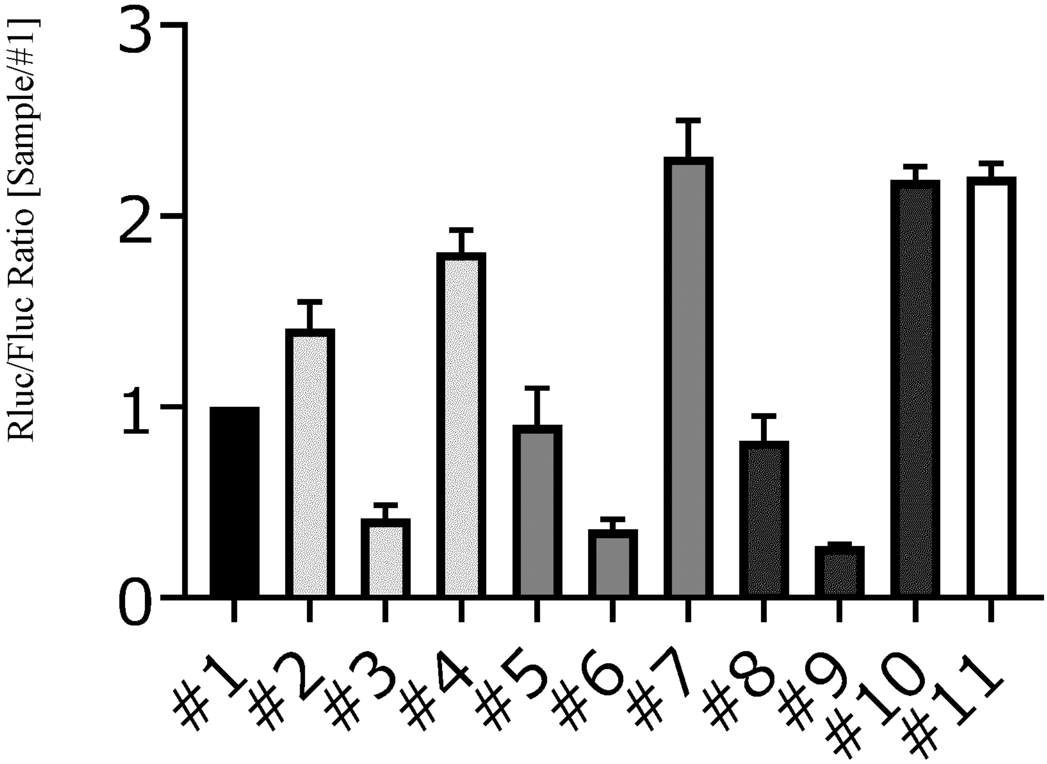


Figure 2: RNA editing 3 days after transfection

[Figure 3]

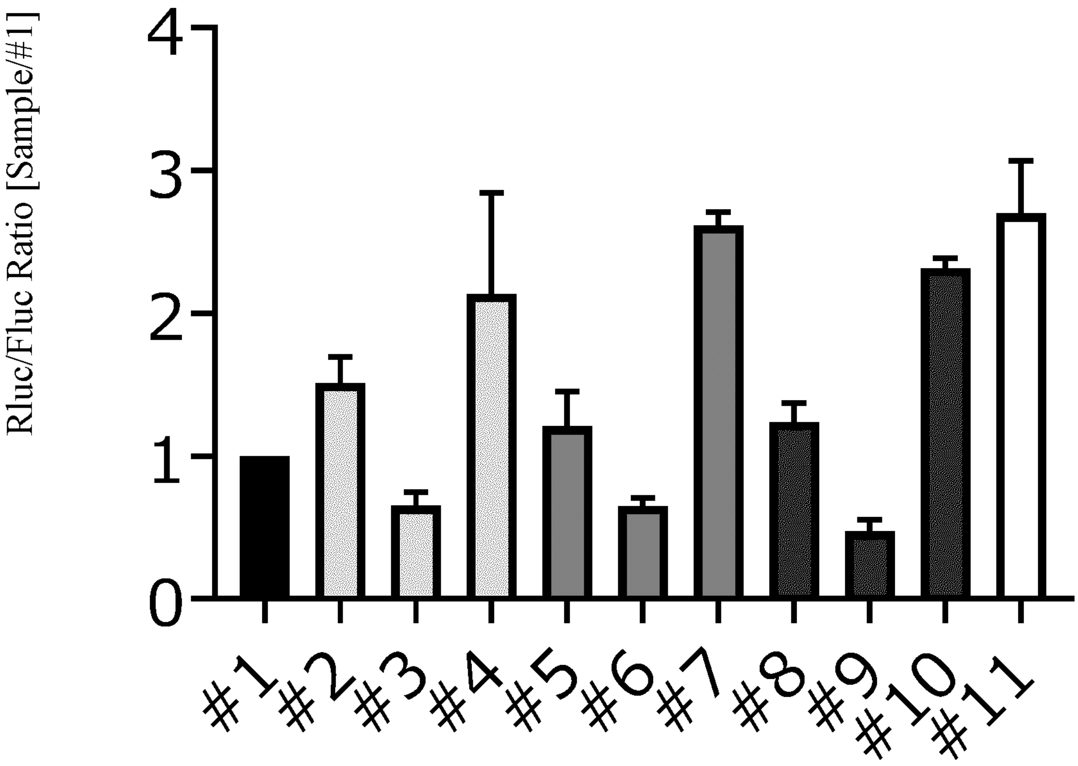


Figure 3: RNA editing 6 days after transfection

[Figure 4]

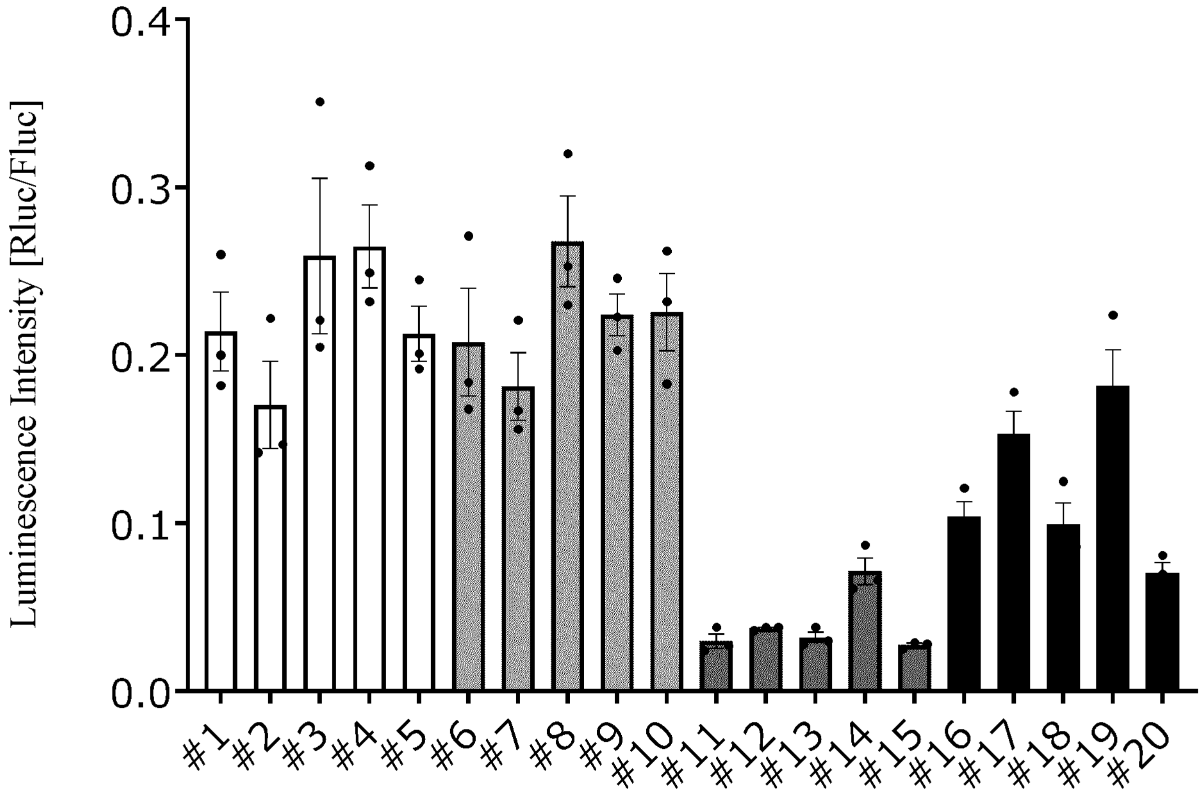


Figure 4: RNA-editing activity

**INTERNATIONAL SEARCH REPORT**

International application No.
PCT/JP2020/029386

A. CLASSIFICATION OF SUBJECT MATTER
C12N 15/11(2006.01)i; A61K 31/7105(2006.01)i; A61K 48/00(2006.01)i; C12N 1/15(2006.01)i; C12N 1/19(2006.01)i; C12N 1/21(2006.01)i; C12N 5/10(2006.01)i; C12N 9/78(2006.01)i; C12N 15/09(2006.01)i; C12N 15/55(2006.01)i; C12N 15/63(2006.01)i
FI: C12N15/11 Z ZNA; A61K31/7105; A61K48/00; C12N1/15; C12N1/19; C12N1/21; C12N5/10; C12N9/78; C12N15/09 100; C12N15/55; C12N15/63 Z
According to International Patent Classification (IPC) or to both national classification and IPC

B. FIELDS SEARCHED
Minimum documentation searched (classification system followed by classification symbols)
C12N15/00-15/90; C12N1/00-9/99; A61K31/33-33/44; A61K48/00

Documentation searched other than minimum documentation to the extent that such documents are included in the fields searched

| | |
|---|---|
| Published examined utility model applications of Japan | 1922-1996 |
| Published unexamined utility model applications of Japan | 1971-2020 |
| Registered utility model specifications of Japan | 1996-2020 |
| Published registered utility model applications of Japan | 1994-2020 |

Electronic data base consulted during the international search (name of data base and, where practicable, search terms used)
JSTPlus/JMEDPlus/JST7580 (JDreamIII), CAplus/MEDLINE/EMBASE/BIOSIS/WPIDS (STN)

C. DOCUMENTS CONSIDERED TO BE RELEVANT

| Category* | Citation of document, with indication, where appropriate, of the relevant passages | Relevant to claim No. |
|---|---|---|
| Y | WO 2019/111957 A1 (FUKUOKA UNIVERSITY) 13.06.2019 (2019-06-13) claims 1, 9, examples, fig. 1, 2B, 3-4, 8, tables 6, 8, 15 | 1-31 |
| Y | NAHAR, Smita et al., "A G-quadruplex motif at the 3' end of sgRNAs improves CRISPR-Cas9 based genome editing efficiency", Chem. Commun., 2018, vol. 54, pp. 2377-2380, abstract, page 2378, left column, lines 11-16, page 2378, right column, lines 12-23, fig. 3 | 1-31 |
| Y | GOOD, P. D. et al., "Expression of small, therapeutic RNAs in human cell nuclei", Gene Ther., 1997, vol. 4, pp. 45-54, abstract, page 46, left column, line 11 to page 48, right column, line 8, fig. 1, 3-4, table 1 | 1-31 |

☒ Further documents are listed in the continuation of Box C. ☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 18 September 2020 (18.09.2020) | 29 September 2020 (29.09.2020) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**

International application No.
PCT/JP2020/029386

C (Continuation). DOCUMENTS CONSIDERED TO BE RELEVANT

| Category* | Citation of document, with indication, where appropriate, of the relevant passages | Relevant to claim No. |
|---|---|---|
| Y | US 2019/0093098 A1 (EBERHARD KARLS UNIVERSITAT TUBINGEN) 28.03.2019 (2019-03-28) claims 1, 10, paragraph [0029], fig. 1, 3 | 1-31 |
| A | WO 2017/010556 A1 (FUKUOKA UNIVERSITY) 19.01.2017 (2017-01-19) | 1-31 |
| A | WETTENGEL, Jacqueline et al., "Harnessing human ADAR2 for RNA repair-Recoding a PINKI mutation rescues mitophagy", Nucleic Acids Res., 2017, vol. 45, pp. 2797-2808, entire text | 1-31 |
| A | US 2003/0148519 A1 (ENGELKE, David R. et al.) 07.08.2003 (2003-08-07) | 1-31 |

Form PCT/ISA/210 (continuation of second sheet) (January 2015)

**INTERNATIONAL SEARCH REPORT**
Information on patent family members

International application no.
PCT/JP2020/029386

| Patent Documents referred in the Report | Publication Date | Patent Family | Publication Date |
|---|---|---|---|
| WO 2019/111957 A1 | 13 Jun. 2019 | JP 2019-97555 A | |
| US 2019/0093098 A1 | 28 Mar. 2019 | WO 2017/050306 A1<br>EP 3353299 A1<br>DE 102015012522 B | |
| WO 2017/010556 A1 | 19 Jan. 2017 | US 2018/0208924 A1<br>EP 3323890 A1 | |
| US 2003/0148519 A1 | 07 Aug. 2003 | US 2009/0042207 A1 | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2017220751 A
- WO 2018041973 A
- WO 2018134301 A
- WO 2016097212 A
- WO 2017050306 A
- DE 102015012522
- WO 2017010556 A
- WO 2019111957 A


### Non-patent literature cited in the description


- *Nature,* 1996, vol. 379, 460-464
- *FEBS Letters,* 2006, vol. 580, 2301-2305
- *Ann. Rev. Biochem.,* 2010, vol. 79, 321-349
- *Science,* 2019, vol. 365, 382-386
- *Nucleic Acids Research,* 2017, vol. 45, 2797-2808
- *Nat. Biotechnol.,* 2019, vol. 37, 133-138
- *Scientific Reports,* 2017, vol. 7, 41478
- *Cell. Mol. LifeSci.,* 2006, vol. 63, 901-908
- *Biol. Cell.,* 2008, vol. 100, 125-138
- *J. Am. Chem. Soc.,* 2002, vol. 124, 10966-10967
- *Nucleic Acids Research,* 2016, vol. 44, e157
- *Cell,* 1989, vol. 59, 871-880
- *EMBO Reports,* 2015, vol. 16, 910-922
- *Chem. Commun.,* 2018, vol. 54, 2377-2380
- *Gene Ther.,* 1997, vol. 4, 45-54
- *Nat. Biotechnol.,* 2002, vol. 20, 505-508
- *Mol. Ther.,* 2003, vol. 7, 237-247
- *Biochemistry,* 2018, vol. 57, 1640-1651
- *RNA,* 2011, vol. 2, 761-771
- *Biophysical Chemistry,* 2013, vol. 180 (181), 110-118
- *ACS Chemical Biology,* 2006, vol. 1, 761-765
- *Biophysical J.,* 2017, vol. 113, 257-267
- *J. Mol. Biol.,* 1970, vol. 48, 443-453
- *Int. J. Biochem. Mol. Biol.,* 2013, vol. 4, 27-40
- *Integr. Biol.,* 2009, vol. 1, 499-505
- *Nucleic Acids Research,* 2016, vol. 44, 9555-9564
- Current Protocols in Molecular Biology. John Wiley & Sons, 1987
- *Trends in Biochemical Sciences,* 2001, vol. 26, 376-384
- *RNA,* 2001, vol. 7, 846-858
- *Nat. Biotechnol.,* 2002, vol. 20, 497-500
- *Nucleic Acids Research,* 2003, vol. 31, e100
- **GREEN, M.R. ; SAMBROOK, J.** Molecular Cloning: A Laboratory Manual. Cold Spring Harbor Laboratory Press, 2012
- *Bioinformatics,* 2009, vol. 25, 3244-3250

SEQUENCE LISTING

<110> Astellas Pharma Inc.
Fukuoka University

<120> A target-editing guide RNA to which a functional sequence is added

<130> G2350WO

<150> JP2019-141875
<151> 2019-08-01

<160> 53

<170> PatentIn version 3.5

<210> 1
<211> 14
<212> RNA
<213> Artificial

<220>
<223> Synthetic RNA

<400> 1
gggguguucgc accu 14

<210> 2
<211> 16
<212> RNA
<213> Artificial

<220>
<223> Synthetic RNA

<400> 2
gggugguucg ccaccu 16

<210> 3
<211> 24
<212> RNA
<213> Artificial

<220>
<223> Synthetic RNA

<400> 3
ggguggaaua uucguauccc accu 24

<210> 4
<211> 34
<212> RNA
<213> Artificial

<220>
<223> Synthetic RNA

<400> 4
ggguggaaua guauauucgu auaguauccc accu 34

<210> 5
<211> 40
<212> RNA
<213> Artificial

<220>
<223> Synthetic RNA

<400> 5
ggguggaaua guauaccauu cgugguauag uaucccaccc 40

<210> 6
<211> 24
<212> RNA
<213> Artificial

<220>
<223> Synthetic RNA

<400> 6
ggguuugggu uuggguuugg guuu 24

<210> 7
<211> 24
<212> RNA
<213> Artificial

<220>
<223> Synthetic RNA

<400> 7
agagggcccu gaagagggcc cuuu 24

<210> 8
<211> 33
<212> RNA
<213> Artificial

<220>
<223> Synthetic RNA

<400> 8
gugcucgcuu cggcagcaca uauacuaguc gac 33

<210> 9
<211> 26
<212> RNA
<213> Artificial

<220>
<223> Synthetic RNA

<400> 9
ucuagagcgg acuucggucc gcuuuu 26

<210> 10

```
<211>  20
<212>  RNA
<213>  Artificial

<220>
<223>  Synthetic RNA

<400>  10
caguccugga aacagggacc                                                   20


<210>  11
<211>  2127
<212>  DNA
<213>  Homo sapiens


<220>
<221>  CDS
<222>  (16)..(2118)

<400>  11
gaattcgccg ccacc atg gat ata gaa gat gaa gaa aac atg agt tcc agc        51
                 Met Asp Ile Glu Asp Glu Glu Asn Met Ser Ser Ser
                 1               5                   10

agc act gat gtg aag gaa aac cgc aat ctg gac aac gtg tcc ccc aag          99
Ser Thr Asp Val Lys Glu Asn Arg Asn Leu Asp Asn Val Ser Pro Lys
        15                  20                  25

gat ggc agc aca cct ggg cct ggc gag ggc tct cag ctc tcc aat ggg         147
Asp Gly Ser Thr Pro Gly Pro Gly Glu Gly Ser Gln Leu Ser Asn Gly
    30                  35                  40

ggt ggt ggt ggc ccc ggc aga aag cgg ccc ctg gag gag ggc agc aat         195
Gly Gly Gly Gly Pro Gly Arg Lys Arg Pro Leu Glu Glu Gly Ser Asn
45                  50                  55                  60

ggc cac tcc aag tac cgc ctg aag aaa agg agg aaa aca cca ggg ccc         243
Gly His Ser Lys Tyr Arg Leu Lys Lys Arg Arg Lys Thr Pro Gly Pro
                65                  70                  75

gtc ctc ccc aag aac gcc ctg atg cag ctg aat gag atc aag cct ggt         291
Val Leu Pro Lys Asn Ala Leu Met Gln Leu Asn Glu Ile Lys Pro Gly
            80                  85                  90

ttg cag tac aca ctc ctg tcc cag act ggg ccc gtg cac gcg cct ttg         339
Leu Gln Tyr Thr Leu Leu Ser Gln Thr Gly Pro Val His Ala Pro Leu
        95                  100                 105

ttt gtc atg tct gtg gag gtg aat ggc cag gtt ttt gag ggc tct ggt         387
Phe Val Met Ser Val Glu Val Asn Gly Gln Val Phe Glu Gly Ser Gly
    110                 115                 120

ccc aca aag aaa aag gca aaa ctc cat gct gct gag aag gcc ttg agg         435
Pro Thr Lys Lys Lys Ala Lys Leu His Ala Ala Glu Lys Ala Leu Arg
125                 130                 135                 140

tct ttc gtt cag ttt cct aat gcc tct gag gcc cac ctg gcc atg ggg         483
Ser Phe Val Gln Phe Pro Asn Ala Ser Glu Ala His Leu Ala Met Gly
                145                 150                 155

agg acc ctg tct gtc aac acg gac ttc aca tct gac cag gcc gac ttc         531
```

Arg Thr Leu Ser Val Asn Thr Asp Phe Thr Ser Asp Gln Ala Asp Phe
160 165 170

cct gac acg ctc ttc aat ggt ttt gaa act cct gac aag gcg gag cct 579
Pro Asp Thr Leu Phe Asn Gly Phe Glu Thr Pro Asp Lys Ala Glu Pro
175 180 185

ccc ttt tac gtg ggc tcc aat ggg gat gac tcc ttc agt tcc agc ggg 627
Pro Phe Tyr Val Gly Ser Asn Gly Asp Asp Ser Phe Ser Ser Ser Gly
190 195 200

gac ctc agc ttg tct gct tcc ccg gtg cct gcc agc cta gcc cag cct 675
Asp Leu Ser Leu Ser Ala Ser Pro Val Pro Ala Ser Leu Ala Gln Pro
205 210 215 220

cct ctc cct gtc tta cca cca ttc cca ccc ccg agt ggg aag aat ccc 723
Pro Leu Pro Val Leu Pro Pro Phe Pro Pro Pro Ser Gly Lys Asn Pro
225 230 235

gtg atg atc ttg aac gaa ctg cgc cca gga ctc aag tat gac ttt ctc 771
Val Met Ile Leu Asn Glu Leu Arg Pro Gly Leu Lys Tyr Asp Phe Leu
240 245 250

tcc gag agc ggg gag agc cat gcc aag agc ttc gtc atg tct gtg gtc 819
Ser Glu Ser Gly Glu Ser His Ala Lys Ser Phe Val Met Ser Val Val
255 260 265

gtg gat ggt cag ttc ttt gaa ggc tcg ggg aga aac aag aag ctt gcc 867
Val Asp Gly Gln Phe Phe Glu Gly Ser Gly Arg Asn Lys Lys Leu Ala
270 275 280

aag gcc cgg gct gcg cag tct gcc ctg gcc gcc att ttt aac ttg cac 915
Lys Ala Arg Ala Ala Gln Ser Ala Leu Ala Ala Ile Phe Asn Leu His
285 290 295 300

ttg gat cag acg cca tct cgc cag cct att ccc agt gag ggt ctt cag 963
Leu Asp Gln Thr Pro Ser Arg Gln Pro Ile Pro Ser Glu Gly Leu Gln
305 310 315

ctg cat tta ccg cag gtt tta gct gac gct gtc tca cgc ctg gtc ctg 1011
Leu His Leu Pro Gln Val Leu Ala Asp Ala Val Ser Arg Leu Val Leu
320 325 330

ggt aag ttt ggt gac ctg acc gac aac ttc tcc tcc cct cac gct cgc 1059
Gly Lys Phe Gly Asp Leu Thr Asp Asn Phe Ser Ser Pro His Ala Arg
335 340 345

aga aaa gtg ctg gct gga gtc gtc atg aca aca ggc aca gat gtt aaa 1107
Arg Lys Val Leu Ala Gly Val Val Met Thr Thr Gly Thr Asp Val Lys
350 355 360

gat gcc aag gtg ata agt gtt tct aca gga aca aaa tgt att aat ggt 1155
Asp Ala Lys Val Ile Ser Val Ser Thr Gly Thr Lys Cys Ile Asn Gly
365 370 375 380

gaa tac atg agt gat cgt ggc ctt gca tta aat gac tgc cat gca gaa 1203
Glu Tyr Met Ser Asp Arg Gly Leu Ala Leu Asn Asp Cys His Ala Glu
385 390 395

ata ata tct cgg aga tcc ttg ctc aga ttt ctt tat aca caa ctt gag 1251
Ile Ile Ser Arg Arg Ser Leu Leu Arg Phe Leu Tyr Thr Gln Leu Glu
400 405 410

```
ctt tac tta aat aac aaa gat gat caa aaa aga tcc atc ttt cag aaa        1299
Leu Tyr Leu Asn Asn Lys Asp Asp Gln Lys Arg Ser Ile Phe Gln Lys
        415                 420                 425

tca gag cga ggg ggg ttt agg ctg aag gag aat gtc cag ttt cat ctg        1347
Ser Glu Arg Gly Gly Phe Arg Leu Lys Glu Asn Val Gln Phe His Leu
    430                 435                 440

tac atc agc acc tct ccc tgt gga gat gcc aga atc ttc tca cca cat        1395
Tyr Ile Ser Thr Ser Pro Cys Gly Asp Ala Arg Ile Phe Ser Pro His
445                 450                 455                 460

gag cca atc ctg gaa gaa cca gca gat aga cac cca aat cgt aaa gca        1443
Glu Pro Ile Leu Glu Glu Pro Ala Asp Arg His Pro Asn Arg Lys Ala
                465                 470                 475

aga gga cag cta cgg acc aaa ata gag tct ggt gag ggg acg att cca        1491
Arg Gly Gln Leu Arg Thr Lys Ile Glu Ser Gly Glu Gly Thr Ile Pro
            480                 485                 490

gtg cgc tcc aat gcg agc atc caa acg tgg gac ggg gtg ctg caa ggg        1539
Val Arg Ser Asn Ala Ser Ile Gln Thr Trp Asp Gly Val Leu Gln Gly
        495                 500                 505

gag cgg ctg ctc acc atg tcc tgc agt gac aag att gca cgc tgg aac        1587
Glu Arg Leu Leu Thr Met Ser Cys Ser Asp Lys Ile Ala Arg Trp Asn
    510                 515                 520

gtg gtg ggc atc cag gga tcc ctg ctc agc att ttc gtg gag ccc att        1635
Val Val Gly Ile Gln Gly Ser Leu Leu Ser Ile Phe Val Glu Pro Ile
525                 530                 535                 540

tac ttc tcg agc atc atc ctg ggc agc ctt tac cac ggg gac cac ctt        1683
Tyr Phe Ser Ser Ile Ile Leu Gly Ser Leu Tyr His Gly Asp His Leu
                545                 550                 555

tcc agg gcc atg tac cag cgg atc tcc aac ata gag gac ctg cca cct        1731
Ser Arg Ala Met Tyr Gln Arg Ile Ser Asn Ile Glu Asp Leu Pro Pro
            560                 565                 570

ctc tac acc ctc aac aag cct ttg ctc agt ggc atc agc aat gca gaa        1779
Leu Tyr Thr Leu Asn Lys Pro Leu Leu Ser Gly Ile Ser Asn Ala Glu
        575                 580                 585

gca cgg cag cca ggg aag gcc ccc aac ttc agt gtc aac tgg acg gta        1827
Ala Arg Gln Pro Gly Lys Ala Pro Asn Phe Ser Val Asn Trp Thr Val
    590                 595                 600

ggc gac tcc gct att gag gtc atc aac gcc acg act ggg aag gat gag        1875
Gly Asp Ser Ala Ile Glu Val Ile Asn Ala Thr Thr Gly Lys Asp Glu
605                 610                 615                 620

ctg ggc cgc gcg tcc cgc ctg tgt aag cac gcg ttg tac tgt cgc tgg        1923
Leu Gly Arg Ala Ser Arg Leu Cys Lys His Ala Leu Tyr Cys Arg Trp
                625                 630                 635

atg cgt gtg cac ggc aag gtt ccc tcc cac tta cta cgc tcc aag att        1971
Met Arg Val His Gly Lys Val Pro Ser His Leu Leu Arg Ser Lys Ile
            640                 645                 650

acc aag ccc aac gtg tac cat gag tcc aag ctg gcg gca aag gag tac        2019
Thr Lys Pro Asn Val Tyr His Glu Ser Lys Leu Ala Ala Lys Glu Tyr
        655                 660                 665
```

```
cag gcc gcc aag gcg cgt ctg ttc aca gcc ttc atc aag gcg ggg ctg        2067
Gln Ala Ala Lys Ala Arg Leu Phe Thr Ala Phe Ile Lys Ala Gly Leu
    670                 675                 680

ggg gcc tgg gtg gag aag ccc acc gag cag gac cag ttc tca ctc acg        2115
Gly Ala Trp Val Glu Lys Pro Thr Glu Gln Asp Gln Phe Ser Leu Thr
685                 690                 695                 700

ccc tgaagatcc                                                          2127
Pro
```

<210> 12
<211> 701
<212> PRT
<213> Homo sapiens

<400> 12

```
Met Asp Ile Glu Asp Glu Glu Asn Met Ser Ser Ser Ser Thr Asp Val
1               5                   10                  15

Lys Glu Asn Arg Asn Leu Asp Asn Val Ser Pro Lys Asp Gly Ser Thr
            20                  25                  30

Pro Gly Pro Gly Glu Gly Ser Gln Leu Ser Asn Gly Gly Gly Gly Gly
        35                  40                  45

Pro Gly Arg Lys Arg Pro Leu Glu Glu Gly Ser Asn Gly His Ser Lys
    50                  55                  60

Tyr Arg Leu Lys Lys Arg Arg Lys Thr Pro Gly Pro Val Leu Pro Lys
65                  70                  75                  80

Asn Ala Leu Met Gln Leu Asn Glu Ile Lys Pro Gly Leu Gln Tyr Thr
                85                  90                  95

Leu Leu Ser Gln Thr Gly Pro Val His Ala Pro Leu Phe Val Met Ser
            100                 105                 110

Val Glu Val Asn Gly Gln Val Phe Glu Gly Ser Gly Pro Thr Lys Lys
        115                 120                 125

Lys Ala Lys Leu His Ala Ala Glu Lys Ala Leu Arg Ser Phe Val Gln
    130                 135                 140

Phe Pro Asn Ala Ser Glu Ala His Leu Ala Met Gly Arg Thr Leu Ser
145                 150                 155                 160

Val Asn Thr Asp Phe Thr Ser Asp Gln Ala Asp Phe Pro Asp Thr Leu
                165                 170                 175
```

```
Phe Asn Gly Phe Glu Thr Pro Asp Lys Ala Glu Pro Pro Phe Tyr Val
            180                 185                 190

Gly Ser Asn Gly Asp Asp Ser Phe Ser Ser Ser Gly Asp Leu Ser Leu
        195                 200                 205

Ser Ala Ser Pro Val Pro Ala Ser Leu Ala Gln Pro Pro Leu Pro Val
    210                 215                 220

Leu Pro Pro Phe Pro Pro Pro Ser Gly Lys Asn Pro Val Met Ile Leu
225                 230                 235                 240

Asn Glu Leu Arg Pro Gly Leu Lys Tyr Asp Phe Leu Ser Glu Ser Gly
                245                 250                 255

Glu Ser His Ala Lys Ser Phe Val Met Ser Val Val Val Asp Gly Gln
            260                 265                 270

Phe Phe Glu Gly Ser Gly Arg Asn Lys Lys Leu Ala Lys Ala Arg Ala
        275                 280                 285

Ala Gln Ser Ala Leu Ala Ala Ile Phe Asn Leu His Leu Asp Gln Thr
    290                 295                 300

Pro Ser Arg Gln Pro Ile Pro Ser Glu Gly Leu Gln Leu His Leu Pro
305                 310                 315                 320

Gln Val Leu Ala Asp Ala Val Ser Arg Leu Val Leu Gly Lys Phe Gly
                325                 330                 335

Asp Leu Thr Asp Asn Phe Ser Ser Pro His Ala Arg Arg Lys Val Leu
            340                 345                 350

Ala Gly Val Val Met Thr Thr Gly Thr Asp Val Lys Asp Ala Lys Val
        355                 360                 365

Ile Ser Val Ser Thr Gly Thr Lys Cys Ile Asn Gly Glu Tyr Met Ser
    370                 375                 380

Asp Arg Gly Leu Ala Leu Asn Asp Cys His Ala Glu Ile Ile Ser Arg
385                 390                 395                 400

Arg Ser Leu Leu Arg Phe Leu Tyr Thr Gln Leu Glu Leu Tyr Leu Asn
                405                 410                 415

Asn Lys Asp Asp Gln Lys Arg Ser Ile Phe Gln Lys Ser Glu Arg Gly
```

420 425 430

Gly Phe Arg Leu Lys Glu Asn Val Gln Phe His Leu Tyr Ile Ser Thr
435 440 445

Ser Pro Cys Gly Asp Ala Arg Ile Phe Ser Pro His Glu Pro Ile Leu
450 455 460

Glu Glu Pro Ala Asp Arg His Pro Asn Arg Lys Ala Arg Gly Gln Leu
465 470 475 480

Arg Thr Lys Ile Glu Ser Gly Glu Gly Thr Ile Pro Val Arg Ser Asn
485 490 495

Ala Ser Ile Gln Thr Trp Asp Gly Val Leu Gln Gly Glu Arg Leu Leu
500 505 510

Thr Met Ser Cys Ser Asp Lys Ile Ala Arg Trp Asn Val Val Gly Ile
515 520 525

Gln Gly Ser Leu Leu Ser Ile Phe Val Glu Pro Ile Tyr Phe Ser Ser
530 535 540

Ile Ile Leu Gly Ser Leu Tyr His Gly Asp His Leu Ser Arg Ala Met
545 550 555 560

Tyr Gln Arg Ile Ser Asn Ile Glu Asp Leu Pro Pro Leu Tyr Thr Leu
565 570 575

Asn Lys Pro Leu Leu Ser Gly Ile Ser Asn Ala Glu Ala Arg Gln Pro
580 585 590

Gly Lys Ala Pro Asn Phe Ser Val Asn Trp Thr Val Gly Asp Ser Ala
595 600 605

Ile Glu Val Ile Asn Ala Thr Thr Gly Lys Asp Glu Leu Gly Arg Ala
610 615 620

Ser Arg Leu Cys Lys His Ala Leu Tyr Cys Arg Trp Met Arg Val His
625 630 635 640

Gly Lys Val Pro Ser His Leu Leu Arg Ser Lys Ile Thr Lys Pro Asn
645 650 655

Val Tyr His Glu Ser Lys Leu Ala Ala Lys Glu Tyr Gln Ala Ala Lys
660 665 670

Ala Arg Leu Phe Thr Ala Phe Ile Lys Ala Gly Leu Gly Ala Trp Val
675 680 685

Glu Lys Pro Thr Glu Gln Asp Gln Phe Ser Leu Thr Pro
690 695 700

<210> 13
<211> 16
<212> DNA
<213> Artificial

<220>
<223> Synthetic DNA

<400> 13
gggtggttcg ccacct 16

<210> 14
<211> 20
<212> DNA
<213> Artificial

<220>
<223> Synthetic DNA

<400> 14
cagtcctgga aacagggacc 20

<210> 15
<211> 24
<212> DNA
<213> Artificial

<220>
<223> Synthetic DNA

<400> 15
gggtttgggt ttgggtttgg gttt 24

<210> 16
<211> 24
<212> DNA
<213> Artificial

<220>
<223> Synthetic DNA

<400> 16
agagggccct gaagagggcc cttt 24

<210> 17
<211> 33
<212> DNA
<213> Artificial

<220>
<223> Synthetic DNA

<400>  17
gtgctcgctt cggcagcaca tatactagtc gac                                    33

<210>  18
<211>  26
<212>  DNA
<213>  Artificial

<220>
<223>  Synthetic DNA

<400>  18
tctagagcgg acttcggtcc gctttt                                            26

<210>  19
<211>  105
<212>  DNA
<213>  Artificial

<220>
<223>  Synthetic DNA

<400>  19
agatctgggg aaggttcagc agctcgaacc aaggggtggt tcgccacctc agtcctggaa       60

acagggaccg ggtttgggtt tgggtttggg ttttttttta agctt                      105

<210>  20
<211>  108
<212>  DNA
<213>  Artificial

<220>
<223>  Synthetic DNA

<400>  20
agatctgggg aaggttcagc agctcgaacc aaggggtggt tcgccacctc agtcctggaa       60

acagggacct ctagagggcc ctgaagaggg cccttttttt ttaagctt                   108

<210>  21
<211>  132
<212>  DNA
<213>  Artificial

<220>
<223>  Synthetic DNA

<400>  21
agatctggga gagggccctg aagagggccc ttttctgaag gttcagcagc tcgaaccaag       60

gggtggttcg ccacctcagt cctggaaaca gggaccgggt ttgggtttgg gtttgggttt      120

tttttaagc tt                                                           132

<210>  22
<211>  85

<212> DNA
<213> Artificial

<220>
<223> Synthetic DNA

<400> 22
agatctgggg aaggttcagc agctcgaacc aaggggtggt tcgccacctg ggtttgggtt 60

tgggtttggg ttttttttta agctt 85

<210> 23
<211> 88
<212> DNA
<213> Artificial

<220>
<223> Synthetic DNA

<400> 23
agatctgggg aaggttcagc agctcgaacc aaggggtggt tcgccacctt ctagagggcc 60

ctgaagaggg cccttttttt ttaagctt 88

<210> 24
<211> 112
<212> DNA
<213> Artificial

<220>
<223> Synthetic DNA

<400> 24
agatctggga gagggccctg aagagggccc ttttctgaag gttcagcagc tcgaaccaag 60

gggtggttcg ccacctgggt ttgggtttgg gtttgggttt ttttttaagc tt 112

<210> 25
<211> 139
<212> DNA
<213> Artificial

<220>
<223> Synthetic DNA

<400> 25
agatctgggt gctcgcttcg gcagcacata tactagtcga cgaaggttca gcagctcgaa 60

ccaaggggtg gttcgccacc tcagtcctgg aaacagggac ctctagagcg gacttcggtc 120

cgcttttttt tttaagctt 139

<210> 26
<211> 120
<212> DNA
<213> Artificial

<220>
<223> Synthetic DNA

<400> 26
agatctgggg tgctcgcttc ggcagcacat atactagtcg acgaaggttc agcagctcga 60

accaaggggt ggttcgccac cttctagagc ggacttcggt ccgctttttt ttttaagctt 120

<210> 27
<211> 85
<212> DNA
<213> Artificial

<220>
<223> Synthetic DNA

<400> 27
agatctgggg aaggttcagc agctcgaacc aaggggtgga atagtatacc attcgtggta 60

tagtatccca cccttttta agctt 85

<210> 28
<211> 81
<212> DNA
<213> Artificial

<220>
<223> Synthetic DNA

<400> 28
agatctgggg aaggttcagc agctcgaacc aaggggtggt tcgccacctc agtcctggaa 60

acaggacct tttttaagct t 81

<210> 29
<211> 61
<212> DNA
<213> Artificial

<220>
<223> Synthetic DNA

<400> 29
agatctgggg aaggttcagc agctcgaacc aaggggtggt tcgccacctt tttttaagct 60

t 61

<210> 30
<211> 105
<212> DNA
<213> Artificial

<220>
<223> Synthetic DNA

<400> 30
agatctgggg aaggttcagc agctcgaacc aaggggtgga atagtatacc attcgtggta 60

tagtatccca ccccagtcct ggaaacaggg accttttta agctt 105

<210> 31
<211> 129
<212> DNA
<213> Artificial

<220>
<223> Synthetic DNA

<400> 31
agatctgggg aaggttcagc agctcgaacc aaggggtgga atagtatacc attcgtggta 60

tagtatccca ccccagtcct ggaaacaggg accgggtttg ggtttgggtt tgggtttttt 120

tttaagctt 129

<210> 32
<211> 135
<212> DNA
<213> Artificial

<220>
<223> Synthetic DNA

<400> 32
agatctgggg aaggttcagc agctcgaacc aaggggtgga atagtatacc attcgtggta 60

tagtatccca ccccagtcct ggaaacaggg acctctagag ggccctgaag agggcccttt 120

ttttttttta agctt 135

<210> 33
<211> 156
<212> DNA
<213> Artificial

<220>
<223> Synthetic DNA

<400> 33
agatctggga gagggccctg aagagggccc ttttctgaag gttcagcagc tcgaaccaag 60

gggtggaata gtataccatt cgtggtatag tatcccaccc cagtcctgga aacagggacc 120

gggtttgggt ttgggtttgg gttttttttt aagctt 156

<210> 34
<211> 163
<212> DNA
<213> Artificial

<220>
<223> Synthetic DNA

<400> 34
agatctgggt gctcgcttcg gcagcacata tactagtcga cgaaggttca gcagctcgaa 60

ccaaggggtg gaatagtata ccattcgtgg tatagtatcc caccccagtc ctggaaacag 120

ggacctctag agcggacttc ggtccgcttt tttttttaag ctt 163

<210> 35
<211> 109
<212> DNA
<213> Artificial

<220>
<223> Synthetic DNA

<400> 35
agatctgggg aaggttcagc agctcgaacc aaggggtgga atagtatacc attcgtggta 60

tagtatccca cccgggtttg ggtttgggtt tgggtttttt tttaagctt 109

<210> 36
<211> 112
<212> DNA
<213> Artificial

<220>
<223> Synthetic DNA

<400> 36
agatctgggg aaggttcagc agctcgaacc aaggggtgga atagtatacc attcgtggta 60

tagtatccca ccctctagag ggccctgaag agggcccttt ttttttaagc tt 112

<210> 37
<211> 136
<212> DNA
<213> Artificial

<220>
<223> Synthetic DNA

<400> 37
agatctggga gagggccctg aagagggccc ttttctgaag gttcagcagc tcgaaccaag 60

gggtggaata gtataccatt cgtggtatag tatcccaccc gggtttgggt ttgggtttgg 120

gttttttttt aagctt 136

<210> 38
<211> 143
<212> DNA
<213> Artificial

<220>
<223> Synthetic DNA

<400> 38
agatctgggt gctcgcttcg gcagcacata tactagtcga cgaaggttca gcagctcgaa 60

ccaaggggtg gaatagtata ccattcgtgg tatagtatcc caccctctag agcggacttc 120

ggtccgcttt tttttttaag ctt 143

<210> 39
<211> 253

<212> DNA
<213> Artificial

<220>
<223> Synthetic DNA

<400> 39
gaattcgagg gcctatttcc catgattcct tcatatttgc atatacgata caaggctgtt 60

agagagataa ttagaattaa tttgactgta aacacaaaga tattagtaca aaatacgtga 120

cgtagaaagt aataatttct tgggtagttt gcagttttaa aattatgttt taaaatggac 180

tatcatatgc ttaccgtaac ttgaaagtat ttcgatttct tggctttata tatcttgtgg 240

aaaggacaga tct 253

<210> 40
<211> 516
<212> DNA
<213> Artificial

<220>
<223> Synthetic DNA

<400> 40
cacgtcgtgc ctcacatcga gcccgtggct agatgcatca tccctgatct gatcggaatg 60

ggtaagtccg gcaagagcgg gaatggctca tatcgcctcc tggatcacta caagtacctc 120

accgcttagt tcgagctgct gaaccttcca aagaaaatca tctttgtggg ccacgactgg 180

ggggcttgtc tggcctttca ctactcctac gagcaccaag acaagatcaa ggccatcgtc 240

catgctgaga gtgtcgtgga cgtgatcgag tcctgggacg agtggcctga catcgaggag 300

gatatcgccc tgatcaagag cgaagagggc gagaaaatgg tgcttgagaa taacttcttc 360

gtcgagacca tgctcccaag caagatcatg cggaaactgg agcctgagga gttcgctgcc 420

tacctggagc cattcaagga gaagggcgag gttagacggc ctaccctctc ctggcctcgc 480

gagatccctc tcgttaaggg aggcaagccc gacgtc 516

<210> 41
<211> 18
<212> DNA
<213> Artificial

<220>
<223> Synthetic DNA

<400> 41
ggtaacgctg cctccagc 18

<210> 42
<211> 20
<212> DNA
<213> Artificial

<220>
<223> Synthetic DNA

<400> 42
tgtagttgcg gacaatctgg 20

<210> 43
<211> 20
<212> DNA
<213> Artificial

<220>
<223> Synthetic DNA

<400> 43
ctcgctgcaa gcaaatgaac 20

<210> 44
<211> 20
<212> DNA
<213> Artificial

<220>
<223> Synthetic DNA

<400> 44
tcgtcccagg actcgatcac 20

<210> 45
<211> 104
<212> DNA
<213> Artificial

<220>
<223> Synthetic DNA

<400> 45
agatcgggtc ttcatggcct tgtgcagccg ctgggtggtt cgccacctca gtcctggaaa 60

cagggaccgg gtttgggttt gggtttgggt tttttttaa gctt 104

<210> 46
<211> 107
<212> DNA
<213> Artificial

<220>
<223> Synthetic DNA

<400> 46
agatcgggtc ttcatggcct tgtgcagccg ctgggtggtt cgccacctca gtcctggaaa 60

cagggacctc tagagggccc tgaagagggc cctttttttt taagctt 107

<210> 47
<211> 138
<212> DNA
<213> Artificial

<220>
<223> Synthetic DNA

<400> 47
agatcgggtg ctcgcttcgg cagcacatat actagtcgac tcttcatggc cttgtgcagc 60

cgctgggtgg ttcgccacct cagtcctgga aacagggacc tctagagcgg acttcggtcc 120

gctttttttt ttaagctt 138

<210> 48
<211> 84
<212> DNA
<213> Artificial

<220>
<223> Synthetic DNA

<400> 48
agatcgggtc ttcatggcct tgtgcagccg ctgggtggaa tagtatacca ttcgtggtat 60

agtatcccac cctttttaa gctt 84

<210> 49
<211> 80
<212> DNA
<213> Artificial

<220>
<223> Synthetic DNA

<400> 49
agatcgggtc ttcatggcct tgtgcagccg ctgggtggtt cgccacctca gtcctggaaa 60

cagggacctt ttttaagctt 80

<210> 50
<211> 60
<212> DNA
<213> Artificial

<220>
<223> Synthetic DNA

<400> 50
agatcgggtc ttcatggcct tgtgcagccg ctgggtggtt cgccaccttt ttttaagctt 60

<210> 51
<211> 25
<212> DNA
<213> Artificial

<220>
<223> Synthetic DNA

<400> 51
ggccgatgct aagaacatta agaag 25

```
<210>  52
<211>  20
<212>  DNA
<213>  Artificial

<220>
<223>  Synthetic DNA

<400>  52
ccacggtagg ctgagaaatg                                                  20


<210>  53
<211>  18
<212>  DNA
<213>  Artificial

<220>
<223>  Synthetic DNA

<400>  53
ctccgatgaa cagggcgc                                                    18
```